# NOUVEAU FASCICULE DE BREVET EUROPEEN

(11) **EP 2 446 038 B2**
(45) Date de publication et mention de la décision concernant l'opposition: **31.10.2018**
(45) Mention de la délivrance du brevet: 08.10.2014
(21) Numéro de dépôt: 10724755.3
(22) Date de dépôt: 03.06.2010
(51) Int. Cl.: C07K 14/475, C07K 16/18, C07K 16/22, G01N 33/574, G01N 33/68

(54) **SEQUENCES, ANTICORPS, PROCEDES ET NECESSAIRES POUR LA DETECTION ET LE DOSAGE IN VITRO DE LA PERIOSTINE, EN VUE DU DIAGNOSTIC DU SUIVI OU DU PRONOSTIC DE PATHOLOGIES OU DE PHENOMENES BIOLOGIQUES IMPLIQUANT LA PERIOSTINE**
SEQUENZEN, ANTIKÖRPER, VERFAHREN ZUM AUFSPÜREN UND ZUR IN VITRO DOSIERUNG VON PERIOSTIN IN DER DIAGNOSE ODER DER PROGNOSE VON PERIOSTIN VERURSACHTEN KRANKHEITEN ODER VON BIOLOGISCHEN PHÄNOMENEN IM ZUSAMMENHANG MIT PERIOSTIN
SEQUENCES, ANTIBODIES, PROCESSES FOR THE DETECTION AND FOR THE IN VITRO DOSAGE OF PERIOSTIN IN THE DIAGNOSTIC OR IN THE PROGNOSTIC OF PERIOSTIN RELATED DISEASES OR BIOLOGICAL PHENOMENS

(30) Priorité: 03.06.2009 FR 0953646
(43) Date de publication de la demande: 02.05.2012
(73) Titulaire: Synarc Sas, 69003 Lyon (FR); INSERM - Institut National de la Santé et de la Recherche Médicale, 75013 Paris (FR)
(72) Inventeur: GARNERO, Patrick, F-30330 Gaujac (FR); CONTIE, Sylvain, F-13090 Aix-en-Provence (FR); ROUSSELOT, Nathalie, F-38138 Les Cotes D'arey (FR); CLEZARDIN, Philippe, F-69002 Lyon (FR)
(74) Mandataire: Cabinet Plasseraud
(86) Numéro de dépôt international: PCT/EP2010/057795
(87) Numéro de publication internationale: WO 2010/139768

(56) Documents cités:
- WO-A-01/57062
- WO-A-03/016471
- WO-A-2005/099743
- CONTIE S ET AL: "Development of a new ELISA for serum periostin: Growth-related changes and effects of bisphosphonate" BONE (NEW YORK), [Online] vol. 44, no. 2, Suppl. S, juin 2009 (2009-06), page S270, XP026136584 & 36TH EUROPEAN SYMPOSIUM ON CALCIFIED TISSUES; VIENNA, AUSTRIA; MAY 23 27, 2009 ISSN: 8756-3282 DOI: 10.1016/j.bone.2009.03.473 [extrait le 2010-02-15]
- COUTU DANIEL L ET AL: "Periostin, a member of a novel family of vitamin K-dependent proteins, is expressed by mesenchymal stromal cells" JOURNAL OF BIOLOGICAL CHEMISTRY, vol. 283, no. 26, juin 2008 (2008-06), pages 17991-18001, XP002598885 ISSN: 0021-9258
- LITVIN JUDITH ET AL: "Expression and function of periostin-isoforms in bone" JOURNAL OF CELLULAR BIOCHEMISTRY, vol. 92, no. 5, 1 août 2004 (2004-08-01), pages 1044-1061, XP002569260 ISSN: 0730-2312
- SASAKI H ET AL: "Novel chemiluminescence assay for the serum periostin levels in woman with preclampsia and in mormotensive pregnant woman" AMERICAN JOURNAL OF OBSTETRICS & GYNECOLOGY, MOSBY, ST LOUIS, MO, US, vol. 186, no. 1, 1 janvier 2002 (2002-01-01), pages 103-108, XP002962762 ISSN: 0002-9378
- YOSHIOKA NAOHISA ET AL: "Suppression of anchorage-independent growth of human cancer cell lines by the TRIF52/periostin/OSF-2 gene" EXPERIMENTAL CELL RESEARCH, vol. 279, no. 1, 10 septembre 2002 (2002-09-10), pages 91-99, XP002569261 ISSN: 0014-4827
- HORIUCHI K ET AL: "Identification and characterization of a novel protein, periostin, with restricted expression to periosteum and periodontal ligament and increased expression by transforming growth factor beta" JOURNAL OF BONE AND MINERAL RESEARCH, AMERICAN SOCIETY FOR BONE AND MINERAL RESEARCH, NEW YORK, NY, US, vol. 14, no. 7, 1 janvier 1999 (1999-01-01), pages 1239-1249, XP003015313 ISSN: 0884-0431
- TAKESHITA S ET AL: "OSTEOBLAST-SPECIFIC FACTOR 2: CLONING OF A PUTATIVE BONE ADHESION PROTEIN WITH HOMOLOGY WITH THE INSECT PROTEIN FASCICLIN I" BIOCHEMICAL JOURNAL, THE BIOCHEMICAL SOCIETY, LONDON, GB, vol. 294, 1 janvier 1993 (1993-01-01), pages 271-278, XP002940428 ISSN: 0264-6021

### SEQUENCE LISTING

<110> SYNARC SAS INSERM-TRANSFERT SA
<120> SEQUENCES, ANTICORPS, PROCEDES ET NECESSAIRES POUR LA DETECTION ET LE DOSAGE IN VITRO DE LA PERIOSTINE, EN VUE DU DIAGNOTIC DU SUIVI OU DU PRONOSTIC DE PATHOLOGIES OU DE PHENOMENES BIOLOGIQUES IMPLIQUANT LA PERIOSTINE
<130> BFF070455
<160> 5
<170> PatentIn version 3.5
<210> 1
   <211> 18
   <212> PRT
   <213> homo sapiens
<400> 1
   Lys Gly Phe Glu Pro Gly Val Thr Asn Ile Leu Lys Thr Thr Gln Gly
   1               5                   10                  15
   Ser Lys
<210> 2
   <211> 17
   <212> PRT
   <213> homo sapiens
<400> 2
   Glu Thr Leu Glu Gly Asn Thr Ile Glu Ile Gly Cys Asp Gly Asp Ser
   1               5                   10                  15
   Ile
<210> 3
   <211> 17
   <212> PRT
   <213> mus musculus
<400> 3
   Glu Ala Ile Thr Gly Gly Ala Val Gly Glu Ala Ile Thr Gly Gly Ala
   1               5                   10                  15
   Val
<210> 4
   <211> 836
   <212> PRT
   <213> homo sapiens
<400> 4
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
           275                 280                 285
   Arg Phe Met Gly Asp Lys Val Ala Ser Glu Ala Leu Met Lys Tyr His
       290                 295                 300
   Ile Leu Asn Thr Leu Gln Cys Ser Glu Ser Ile Met Gly Gly Ala Val
   305                 310                 315                 320
   Phe Glu Thr Leu Glu Gly Asn Thr Ile Glu Ile Gly Cys Asp Gly Asp
                   325                 330                 335
   Ser Ile Thr Val Asn Gly Ile Lys Met Val Asn Lys Lys Asp Ile Val
               340                 345                 350
   Thr Asn Asn Gly Val Ile His Leu Ile Asp Gln Val Leu Ile Pro Asp
           355                 360                 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
                   405                 410                 415
   Thr Leu Ser Met Val Gln Arg Leu Leu Lys Leu Ile Leu Gln Asn His
               420                 425                 430
   Ile Leu Lys Val Lys Val Gly Leu Asn Glu Leu Tyr Asn Gly Gln Ile
           435                 440                 445
   Leu Glu Thr Ile Gly Gly Lys Gln Leu Arg Val Phe Val Tyr Arg Thr
       450                 455                 460
   Ala Val Cys Ile Glu Asn Ser Cys Met Glu Lys Gly Ser Lys Gln Gly
   465                 470                 475                 480
   Arg Asn Gly Ala Ile His Ile Phe Arg Glu Ile Ile Lys Pro Ala Glu
                   485                 490                 495
   Lys Ser Leu His Glu Lys Leu Lys Gln Asp Lys Arg Phe Ser Thr Phe
               500                 505                 510
   Leu Ser Leu Leu Glu Ala Ala Asp Leu Lys Glu Leu Leu Thr Gln Pro
   515 520 525
   Gly Asp Trp Thr Leu Phe Val Pro Thr Asn Asp Ala Phe Lys Gly Met
   530 535 540
   Thr Ser Glu Glu Lys Glu Ile Leu Ile Arg Asp Lys Asn Ala Leu Gln
   545 550 555 560
   Asn Ile Ile Leu Tyr His Leu Thr Pro Gly Val Phe Ile Gly Lys Gly
   565 570 575
   Phe Glu Pro Gly Val Thr Asn Ile Leu Lys Thr Thr Gln Gly Ser Lys
   580 585 590
   Ile Phe Leu Lys Glu Val Asn Asp Thr Leu Leu Val Asn Glu Leu Lys
   595 600 605
   Ser Lys Glu Ser Asp Ile Met Thr Thr Asn Gly Val Ile His Val Val
   610 615 620
   Asp Lys Leu Leu Tyr Pro Ala Asp Thr Pro Val Gly Asn Asp Gln Leu
   625 630 635 640
   Leu Glu Ile Leu Asn Lys Leu Ile Lys Tyr Ile Gln Ile Lys Phe Val
   645 650 655
   Arg Gly Ser Thr Phe Lys Glu Ile Pro Val Thr Val Tyr Thr Thr Lys
   660 665 670
   Ile Ile Thr Lys Val Val Glu Pro Lys Ile Lys Val Ile Glu Gly Ser
   675 680 685
   Leu Gln Pro Ile Ile Lys Thr Glu Gly Pro Thr Leu Thr Lys Val Lys
   690 695 700
   Ile Glu Gly Glu Pro Glu Phe Arg Leu Ile Lys Glu Gly Glu Thr Ile
   705 710 715 720
   Thr Glu Val Ile His Gly Glu Pro Ile Ile Lys Lys Tyr Thr Lys Ile
   725 730 735
   Ile Asp Gly Val Pro Val Glu Ile Thr Glu Lys Glu Thr Arg Glu Glu
   740 745 750
   Arg Ile Ile Thr Gly Pro Glu Ile Lys Tyr Thr Arg Ile Ser Thr Gly
   755 760 765
   Gly Gly Glu Thr Glu Glu Thr Leu Lys Lys Leu Leu Gln Glu Glu Val
       770                 775                 780
   Thr Lys Val Thr Lys Phe Ile Glu Gly Gly Asp Gly His Leu Phe Glu
   785                 790                 795                 800
   Asp Glu Glu Ile Lys Arg Leu Leu Gln Gly Asp Thr Pro Val Arg Lys
                   805                 810                 815
   Leu Gln Ala Asn Lys Lys Val Gln Gly Ser Arg Arg Arg Leu Arg Glu
               820                 825                 830
   Gly Arg Ser Gln
           835
<210> 5
   <211> 838
   <212> PRT
   <213> mus musculus
<400> 5
   Met Val Pro Leu Leu Pro Leu Tyr Ala Leu Leu Leu Leu Phe Leu Cys
   1               5                   10                  15
   Asp Ile Asn Pro Ala Asn Ala Asn Ser Tyr Tyr Asp Lys Val Leu Ala
               20                  25                  30
   His Ser Arg Ile Arg Gly Arg Asp Gln Gly Pro Asn Val Cys Ala Leu
           35                  40                  45
   Gln Gln Ile Leu Gly Thr Lys Lys Lys Tyr Phe Ser Ser Cys Lys Asn
       50                  55                  60
   Trp Tyr Gln Gly Ala Ile Cys Gly Lys Lys Thr Thr Val Leu Tyr Glu
   65                  70                  75                  80
   Cys Cys Pro Gly Tyr Met Arg Met Glu Gly Met Lys Gly Cys Pro Ala
                   85                  90                  95
   Val Met Pro Ile Asp His Val Tyr Gly Thr Leu Gly Ile Val Gly Ala
               100                 105                 110
   Thr Thr Thr Gln His Tyr Ser Asp Val Ser Lys Leu Arg Glu Glu Ile
           115                 120                 125
   Glu Gly Lys Gly Ser Tyr Thr Tyr Phe Ala Pro Ser Asn Glu Ala Trp
       130                 135                 140
   Glu Asn Leu Asp Ser Asp Ile Arg Arg Gly Leu Glu Asn Asn Val Asn
   145                 150                 155                 160
   Val Glu Leu Leu Asn Ala Leu His Ser His Met Val Asn Lys Arg Met
   165 170 175
   Leu Thr Lys Asp Leu Lys His Gly Met Val Ile Pro Ser Met Tyr Asn
   180 185 190
   Asn Leu Gly Leu Phe Ile Asn His Tyr Pro Asn Gly Val Val Thr Val
   195 200 205
   Asn Cys Ala Arg Val Ile His Gly Asn Gln Ile Ala Thr Asn Gly Val
   210 215 220
   Val His Val Ile Asp Arg Val Leu Thr Gln Ile Gly Thr Ser Ile Gln
   225 230 235 240
   Asp Phe Leu Glu Ala Glu Asp Asp Leu Ser Ser Phe Arg Ala Ala Ala
   245 250 255
   Ile Thr Ser Asp Leu Leu Glu Ser Leu Gly Arg Asp Gly His Phe Thr
   260 265 270
   Leu Phe Ala Pro Thr Asn Glu Ala Phe Glu Lys Leu Pro Arg Gly Val
   275 280 285
   Leu Glu Arg Ile Met Gly Asp Lys Val Ala Ser Glu Ala Leu Met Lys
   290 295 300
   Tyr His Ile Leu Asn Thr Leu Gln Cys Ser Glu Ala Ile Thr Gly Gly
   305 310 315 320
   Ala Val Phe Glu Thr Met Glu Gly Asn Thr Ile Glu Ile Gly Cys Glu
   325 330 335
   Gly Asp Ser Ile Ser Ile Asn Gly Ile Lys Met Val Asn Lys Lys Asp
   340 345 350
   Ile Val Thr Lys Asn Gly Val Ile His Leu Ile Asp Glu Val Leu Ile
   355 360 365
   Pro Asp Ser Ala Lys Gln Val Ile Glu Leu Ala Gly Lys Gln Gln Thr
   370 375 380
   Thr Phe Thr Asp Leu Val Ala Gln Leu Gly Leu Ala Ser Ser Leu Lys
   385 390 395 400
   Pro Asp Gly Glu Tyr Thr Leu Leu Ala Pro Val Asn Asn Ala Phe Ser
   405 410 415
   Asp Asp Thr Leu Ser Met Asp Gln Arg Leu Leu Lys Leu Ile Leu Gln
               420                 425                 430
   Asn His Ile Leu Lys Val Lys Val Gly Leu Ser Asp Leu Tyr Asn Gly
           435                 440                 445
   Gln Ile Leu Glu Thr Ile Gly Gly Lys Gln Leu Arg Val Phe Val Tyr
       450                 455                 460
   Arg Thr Ala Ile Cys Ile Glu Asn Ser Cys Met Val Arg Gly Ser Lys
   465                 470                 475                 480
   Gln Gly Arg Asn Gly Ala Ile His Ile Phe Arg Glu Ile Ile Gln Pro
                   485                 490                 495
   Ala Glu Lys Ser Leu His Asp Lys Leu Arg Gln Asp Lys Arg Phe Ser
               500                 505                 510
   Ile Phe Leu Ser Leu Leu Glu Ala Ala Asp Leu Lys Asp Leu Leu Thr
           515                 520                 525
   Gln Pro Gly Asp Trp Thr Leu Phe Ala Pro Thr Asn Asp Ala Phe Lys
       530                 535                 540
   Gly Met Thr Ser Glu Glu Arg Glu Leu Leu Ile Gly Asp Lys Asn Ala
   545                 550                 555                 560
   Leu Gln Asn Ile Ile Leu Tyr His Leu Thr Pro Gly Val Tyr Ile Gly
                   565                 570                 575
   Lys Gly Phe Glu Pro Gly Val Thr Asn Ile Leu Lys Thr Thr Gln Gly
               580                 585                 590
   Ser Lys Ile Tyr Leu Lys Gly Val Asn Glu Thr Leu Leu Val Asn Glu
           595                 600                 605
   Leu Lys Ser Lys Glu Ser Asp Ile Met Thr Thr Asn Gly Val Ile His
       610                 615                 620
   Val Val Asp Lys Leu Leu Tyr Pro Ala Asp Ile Pro Val Gly Asn Asp
   625                 630                 635                 640
   Gln Leu Leu Glu Leu Leu Asn Lys Leu Ile Lys Tyr Ile Gln Ile Lys
                   645                 650                 655
   Phe Val Arg Gly Ser Thr Phe Lys Glu Ile Pro Met Thr Val Tyr Thr
               660                 665                 670
   Thr Lys Ile Ile Thr Lys Val Val Glu Pro Lys Ile Lys Val Ile Gln
           675                 680                 685
   Gly Ser Leu Gln Pro Ile Ile Lys Thr Glu Gly Pro Ala Met Thr Lys
       690                 695                 700
   Ile Gln Ile Glu Gly Asp Pro Asp Phe Arg Leu Ile Lys Glu Gly Glu
   705                 710                 715                 720
   Thr Val Thr Glu Val Ile His Gly Glu Pro Val Ile Lys Lys Tyr Thr
                   725                 730                 735
   Lys Ile Ile Asp Gly Val Pro Val Glu Ile Thr Glu Lys Gln Thr Arg
               740                 745                 750
   Glu Glu Arg Ile Ile Thr Gly Pro Glu Ile Lys Tyr Thr Arg Ile Ser
           755                 760                 765
   Thr Gly Gly Gly Glu Thr Gly Glu Thr Leu Gln Lys Phe Leu Gln Lys
       770                 775                 780
   Glu Val Ser Lys Val Thr Lys Phe Ile Glu Gly Gly Asp Gly His Leu
   785                 790                 795                 800
   Phe Glu Asp Glu Glu Ile Lys Arg Leu Leu Gln Gly Asp Thr Pro Ala
                   805                 810                 815
   Lys Lys Ile Pro Ala Asn Lys Arg Val Gln Gly Pro Arg Arg Arg Ser
               820                 825                 830
   Arg Glu Gly Arg Ser Gln
           835

## Description

### DOMAINE TECHNIQUE DE L'INVENTION

Le domaine de l'invention est celui des outils et des méthodes de détection et de dosage in vitro de la périostine, notamment en vue du diagnostic, du suivi (évaluation) et du pronostic de pathologies ou de phénomènes biologiques impliquant la périostine. Concernant les pathologies, il s'agit notamment de maladies osseuses comme l'ostéoporose, de maladies ostéo-articulaires comme l'arthrose et la polyarthrite rhumatoïde mais également de certains cancers à forts pouvoirs métastatiques comme le cancer du sein, le cancer de la prostate. Concernant les phénomènes biologiques non pathologiques impliquant la périostine, il peut s'agir de la modification ou le remodelage du périoste et du cartilage articulaire, ceci étant lié par exemple à la croissance de l'individu, au changement de son activité motrice ou à des changements hormonaux.

Plus précisément, l'invention vise des séquences protéiques de détection in vitro de la périostine, des moyens de reconnaissance de ces séquences (de préférence des anticorps), des procédés d'obtention de ces moyens de reconnaissance, des procédés et des nécessaires de détection et de dosage mettant en oeuvre ces moyens, en vue d'applications du type diagnostic, suivi et pronostic des maladies susvisées, notamment les maladies osseuses, ostéo-articulaires et des cancers métastatiques.

L'invention concerne également les séquences nucléiques codant pour ces séquences protéiques de détection.

### ART ANTERIEUR ET PROBLEME TECHNIQUE

Le squelette est constitué d'os de différentes natures : les os longs, les os courts, les os plats et les os intermédiaires. Ils ont des rôles physiologiques essentiels. En effet, outre un rôle de charpente et de protection des organes internes, ils sont impliqués, entre autres, dans la formation des cellules sanguines et immunitaires, dans le métabolisme calcique et minéral et dans la croissance de l'individu. Ils sont également impliqués dans le système locomoteur grâce aux muscles s'insérant sur eux et au système articulaire.

Le périoste est la membrane entourant la face externe des os plats, ainsi que des os longs au niveau de leurs diaphyses et métaphyses. Il a plusieurs particularités et fonctions. En effet, il est le « passage » par lequel les systèmes vasculaire et nerveux vont atteindre l'intérieur de l'os. Il sert d'interface avec les éléments extra-osseux tels que les tendons et les ligaments. Il réagit aux contraintes mécaniques (transmises par les ligaments et tendons, forces de torsion, de compression) par des adaptations structurales. Par ailleurs, il est le siège de l'ostéogenèse périphérique qui est déterminante pour la croissance du diamètre externe des os longs et donc pour leur solidité. Le métabolisme du périoste est régulé par des signaux d'origine endogène (hormones) ou exogène (agents thérapeutiques). L'os est une structure qui évolue au cours de la vie de l'individu. Cette évolution est liée à un équilibre physiologique (homéostasie) entre l'activité de deux types cellulaires : d'une part, les cellules assurant la formation et la minéralisation de l'os (les ostéoblastes, OBL), et, d'autre part, les cellules assurant la dégradation de l'os (les ostéoclastes, OCL). Certains événements, pathologiques ou non, peuvent perturber cet équilibre physiologique dans l'os, tels que certains cancers ou une variation du taux d'oestrogènes.

Certains cancers ont un fort pouvoir métastatique comme le cancer du sein ou de la prostate. Les métastases osseuses sont des complications fréquentes de ces cancers. Elles sont responsables, sur le plan clinique, de fractures, d'hypercalcémie et de douleurs qui peuvent engager le pronostic vital ou aggraver rapidement la qualité de vie des patients. Ces métastases modifient dramatiquement l'homéostasie de l'os avec un impact principalement ostéolytique dans le cancer du sein ou ostéoblastique dans les cas de cancer prostatique.

L'une des protéines majeures constituant le périoste et impliquée dans le métabolisme de l'os est la périostine. Cette protéine fait partie de la matrice extracellulaire osseuse. Elle est sécrétée majoritairement par les précurseurs des OBL et les OBL mais aussi par les OCL et a un rôle dans l'adhésion, le recrutement et la différenciation des progéniteurs ostéoblastiques (Horiuchi K, Kudo A. Identification and characterization of a novel protein, Periostin, with restricted expression to periosteum and periodontal ligament and increased expression by transforming growth factor β. J Bone Miner Res. 1999;14(7): 1239-1249 et Litvin J, Safadi FF. Expression and function of periostin-isoforms in bone. J Cell Biochem. 2004; 92: 1044-1061). Son expression est activée par divers facteurs de croissance. La périostine peut subir des modifications post-traductionnelles comme par exemple la gamma-carboxylation (Coutu DL, Wu JH, Monette A, Rivard GE, Blostein MD, Galipeau J. Periostin : a member of a novel family of vitamin k-dependent proteins is expressed by mesenchymal stromal cells. J. Biol. Chem. 2008 Apr 30). Cette modification entraîne, d'une part, un changement de conformation tridimensionnelle et, d'autre part, un gain d'affinité de la périostine vis-à-vis de la matrice minérale.

Connaissant l'implication de cette protéine dans le métabolisme osseux, il a été envisagé de l'utiliser pour le diagnostic, le pronostic et le suivi de pathologies liées à l'os.

Le brevet EP-B-1442295 divulgue ainsi une méthode de diagnostic de ces pathologies exploitant le fait que les taux sériques de périostine sont élevés dans les cas de cancer du sein et de pré-éclampsie. Cette méthode se base sur l'utilisation de différentes isoformes de la périostine et des anticorps les reconnaissant spécifiquement. Ces derniers servent à doser dans un échantillon toutes les isoformes de la périostine ou une isoforme particulière par une technique d'ELISA en sandwich. Cette méthode n'est cependant pas économique car elle nécessite la génération de tout un panel d'anticorps pour doser la périostine totale et présente un risque dans la fiabilité du dosage étant donnés les différents problèmes de réactions croisées possibles et par conséquent le manque de spécificité. En outre, cette méthode permet certes le diagnostic mais ne donne pas accès au pronostic et au suivi des patients ayant reçu un traitement médical et/ou chirurgical pour des pathologies impliquant des isoformes particulières de la périostine.

Par ailleurs, la périostine a été utilisée dans le diagnostic de pathologies autres que celles liées à l'os. La demande de brevet WO-A-2007/077934 décrit la réalisation d'anticorps spécifiques de la périostine ainsi que leur utilisation dans un procédé de détection de cette protéine pour le diagnostic de pathologies telles que des pathologies cardiaques, l'anévrisme, les cancers hautement métastatiques. Dans ce but, il est fabriqué en réalité une multitude d'anticorps, chacun étant spécifique d'une isoforme particulière de la périostine. Ceci présente les mêmes inconvénients économiques (coût, temps, spécificité) que le brevet précité EP-B-1442295.

La demande de brevet WO-A-2007/096142 décrit une méthode d'identification des maladies ou des conditions associées à une néo-vascularisation dans un tissu, ceci grâce à la détection d'au moins une protéine spécifique dudit tissu et par l'utilisation d'anticorps spécifiques de cette(ces) protéine(s). Une des protéines utilisées dans ce but est la périostine sous forme de précurseur, ses isoformes et de nouveaux variants protéiques. Les maladies visées sont la dégénérescence maculaire, l'arthrite, l'athérosclérose et/ou les tumeurs, en particulier les tumeurs du rein. Ainsi, est générée une multitude d'anticorps dirigés contre des antigènes de séquences protéiques différentes avec les mêmes inconvénients qu'exposés précédemment.

La demande de brevet WO-A-2005/019471 décrit une méthode pour diagnostiquer les maladies liées au PLF (periotin like factor), une isoforme particulière de la périostine. Cette méthode se base sur l'utilisation d'un test immuno-enzymatique avec un anticorps spécifique de cette isoforme, l'épitope reconnu par ledit anticorps se trouvant dans la partie variable de la périostine. Cette méthode ne permet de doser qu'une forme particulière de périostine et non la périostine totale exprimée.

### OBJECTIFS PRINCIPAUX DE L'INVENTION

Au vu de cet art antérieur, il était donc souhaitable de trouver de nouveaux moyens spécifiques, simples, fiables, précis et économiques pour déterminer in vitro le taux de périostine totale exprimée ainsi que le taux de périostine sous-carboxylée (totalement ou partiellement) et de périostine carboxylée de manière directe ou indirecte, afin de permettre un diagnostic, un suivi, un pronostic et le traitement des pathologies impliquant la périostine et/ou pour évaluer des phénomènes biologiques non pathologiques tels que l'activité métabolique de l'os. Aussi, les inventeurs ont-ils développé une séquence de détection de la périostine totale ainsi qu'un moyen de reconnaissance de cette séquence de détection permettant de doser in vitro la périostine totale, voire la périostine carboxylée et la périostine sous-carboxylée, dans les fluides biologiques.

Un autre objectif de l'invention est de fournir des moyens et des procédés de détection et de dosage de la périostine totale, sans distinction des différentes isoformes, ainsi que de la périostine sous-carboxylée et indirectement ou directement (par l'utilisation spécifique d'anticorps reconnaissant la forme carboxylée uniquement de la périostine) de la périostine carboxylée, en vue de visualiser et/ou de comprendre des phénomènes biologiques impliquant la périostine, mais aussi en vue d'améliorer le diagnostic précoce, le traitement, le suivi et le pronostic de maladies impliquant la périostine.

Un autre objectif de la présente invention est de fournir des procédés économiques et performants d'étude des phénomènes biologiques impliquant la périostine, ainsi que de diagnostic précoce, de suivi de traitement et de pronostic de maladies impliquant la périostine.

Un autre objectif de la présente invention est de fournir des nécessaires de détection et de dosage de la périostine utiles dans les procédés susvisés.

### BREVE DESCRIPTION DE L'INVENTION

Après de longues recherches et grâce à l'utilisation d'outils sophistiqués et à des modélisations avec des critères de sélection particuliers (exposition, immunogénicité...), les inventeurs ont atteint ces objectifs en développant une nouvelle séquence peptidique de détection de la périostine totale. Ce moyen de détection permet de détecter la périostine totale sans distinction des différentes isoformes de cette protéine et indépendamment des clivages qui se produisent fréquemment aux extrémités des protéines. En effet, à l'aide de modélisations, les inventeurs ont pu mettre en évidence dans la partie constante de la périostine, une séquence peptidique restreinte et très exposée. Cette séquence de détection correspond à une séquence peptidique donnée et ses homologues. Cette séquence de détection permet de détecter de façon simple, précoce, fiable, performante, spécifique et économique des phénomènes biologiques ou des pathologies impliquant la périostine.

La présente invention concerne donc une séquence peptidique SP de détection de la périostine, caractérisée en ce qu'elle comprend ou est constituée par une séquence de 6 à 30 (de préférence 15 à 26) acides aminés et inclut la séquence peptidique SEQ ID N°1, SEQ ID N°2 ou SEQ ID N°3 ou une séquence peptidique présentant un degré d'homologie supérieur ou égal à 80%, de préférence supérieur ou égal à 85% vis-à-vis d'au moins l'une des séquences SEQ ID N°1, SEQ ID N°2 et SEQ ID N°3.

La séquence nucléique codant la séquence de détection SP forme un autre objet de l'invention. Il est également divulgué:
- Des moyens de détection de la périostine, comprenant un outil de reconnaissance spécifique de SP, constitué par au moins un anticorps anti-périostine et/ou au moins l'un de ses fragments fonctionnels (Ac anti-périostine), spécifiques de SEQ ID N°1, SEQ ID N°2, SEQ ID N°3 ou d'une séquence peptidique présentant un degré d'homologie supérieur ou égal à 80%, de préférence supérieur ou égal à 85% vis-à-vis d'au moins l'une des séquences SEQ ID N°1, SEQ ID N°2 et SEQ ID N°3.
- Un procédé de production de ces moyens de détection de la périostine, en particulier d'Ac anti-périostine, caractérisé en ce qu'il consiste essentiellement à :
   a. mettre en oeuvre au moins un antigène représenté par une séquence peptidique SP de détection de la périostine constituée par 6 à 30 acides aminés et incluant tout ou partie de la séquence peptidique SEQ ID N°1, SEQ ID N°2 ou SEQ ID N°3 ou d'une séquence peptidique présentant un degré d'homologie supérieur ou égal à 80%, de préférence supérieur ou égal à 85% vis-à-vis d'au moins l'une des séquences SEQ ID N°1, SEQ ID N°2 et SEQ ID N°3,
   b. coupler ledit antigène à au moins une molécule porteuse,
   c. récupérer du sang/plasma/sérum d'un animal auquel a été préalablement injecté le couple antigène-molécule porteuse,
   d. sélectionner les anticorps spécifiques de SP en mettant le sérum/plasma récupéré à l'étape d) avec des antigènes SP,
   e. éventuellement purifier les anticorps.
- Un procédé de détection et éventuellement de dosage de la périostine totale dans un échantillon biologique, caractérisé en ce qu'il consiste essentiellement à :
   I. mettre en oeuvre un échantillon biologique,
   II. mettre en présence ledit échantillon biologique avec les moyens de détection susvisés,
   III. mettre en évidence les éventuels complexes *Ac anti-périostine*/*SP* formés;
   IV. éventuellement doser ces complexes *Ac anti-périostine*/*SP* formés pour en déduire la concentration en périostine dans l'échantillon.
- Un procédé de détection et éventuellement de dosage de la périostine sous-carboxylée (*psc*), à distinguer de la périostine carboxylée *(pc)*, caractérisé en ce qu'il consiste essentiellement à:
   I*^{psc}*.- mettre en oeuvre un échantillon biologique,
   *I^{psc}*.1- mettre en présence dans un milieu donné ledit échantillon biologique avec un support apte à fixer la *pc* de préférence à la *psc* ou inversement, ledit support étant :
      - avantageusement un support ayant une affinité vis-à-vis du *pc* supérieure à celle vis-à-vis du *psc,*
      - et, de manière plus avantageuse, un support comprenant de l'hydroxyapatite,
      - et, de manière encore plus avantageuse, un support comprenant de la matrice minérale osseuse,
   I*^{psc}*.2- éventuellement agiter ledit milieu,
   I*^{psc}*.3- une fois la fixation effectuée, séparer (de préférence par gradient de densité, et, plus préférentiellement encore, par centrifugation) le support de fixation chargé *en pc* ou en *psc* (de préférence en *pc*) du reste du milieu,
   II*^{psc}*. mettre en présence le support de fixation chargé en *pc* ou en *psc* (de préférence en *pc*) ou le reste du milieu avec les susdits moyens de détection, cette dernière alternative étant préférée,
   III*^{psc}*. mettre en évidence les éventuels complexes *Ac anti-périostine*/*SP* formés;
   IV*^{psc}*. doser ces complexes *Ac anti-périostine*/*SP* formés pour en déduire la concentration en périostine *pc* dans le support de fixation ou en périostine *psc* dans le reste du milieu, cette dernière alternative étant préférée, et *in fine* dans l'échantillon.
- Un procédé de mise en évidence et d'évaluation d'un phénomène biologique non pathologique impliquant la périostine, ou de diagnostic, de suivi ou de pronostic d'une pathologie impliquant la périostine, ou de détermination de l'efficacité d'un médicament adapté au traitement d'une pathologie impliquant la périostine, caractérisé en ce qu'il consiste à mettre en oeuvre le susdit procédé de détection et éventuellement de dosage de la périostine totale dans un échantillon biologique.
- Un nécessaire de détection et de dosage in vitro de la périostine, dans un échantillon biologique, caractérisé en ce qu'il comprend les susdits moyens de détection de la périostine, des réactifs et du matériel pour la mise en évidence, voire le dosage, des éventuels complexes *Ac anti-périostine*/*SP* formés, selon les étapes III, voire IV, du procédé susvisé de détection et éventuellement de dosage de la périostine totale, et une notice de mise en oeuvre.
- Un nécessaire de détection et de dosage de la périostine sous-carboxylée *psc,* caractérisé en ce qu'il comprend les susdits moyens de détection de la périostine, des réactifs et du matériel pour la mise en évidence, voire le dosage, des éventuels complexes *Ac anti-périostine*/*SP* formés selon les étapes III, voire IV, du procédé susvisé de détection et éventuellement de dosage de la périostine totale, un support pour la mise en oeuvre des étapes I*^{psc}*.et II*^{pcs}* du procédé susvisé procédé de détection et éventuellement de dosage de la périostine sous-carboxylée *(psc),* c'est-à-dire un support apte à fixer la *pc* de préférence à la psc ou inversement, ledit support étant :
   - avantageusement un support ayant une affinité vis-à-vis du *pc* supérieure à celle vis-à-vis du *psc,*
   - et, de manière plus avantageuse, un support comprenant de l'hydroxyapatite ou du sulfate de barium,
   - et, de manière encore plus avantageuse, un support comprenant de la matrice minérale osseuse comprenant de l'hydroxyapatite sous forme de cristaux.
   et une notice de mise en oeuvre.

### DESCRIPTION DETAILLEE DE L'INVENTION

La périostine est une protéine de la matrice extracellulaire osseuse. Elle se compose d'une partie principale constante et d'une partie C-terminale plus courte dont la composition en acides aminés est variable. Cette variabilité mène donc à différentes isoformes.

Au sens de l'invention, on désigne *e.g.* par "périostine", toutes les isoformes connues de la périostine sans distinction, quelles que soient les modifications comme les modifications post-traductionnelles (carboxylation, glycosylation, acétylation...) ou autres. On distingue ainsi dans le présent exposé, la périostine ou périostine totale, la périostine carboxylée -ci-après dénommée *"pc"-*, la périostine sous-carboxylée ou décarboxylée-ci-après dénommée *"psc"*-... A défaut de précision, le terme « périostine » désigne la périostine totale.

Les termes "peptide", "polypeptide" et "protéine" seront utilisés de façon interchangeable et ils signifient n'importe quelle chaîne d'acides aminés liés entre eux indépendamment de la longueur de la chaîne et de ses modifications post-traductionnelles.

### Séquences SP de détection

Les séquences de détection selon l'invention SP sont constituées de 6 à 30 (de préférence 15 à 26) acides aminés et incluent la séquence peptidique SEQ ID N°1, SEQ ID N°2 ou SEQ ID N°3 ou une séquence peptidique homologue.

Dans un mode de réalisation particulier, les séquences de détection SP selon l'invention sont constituées de 6 à 30 (de préférence 15 à 26) acides aminés et incluent tout ou partie de la séquence peptidique SEQ ID N°1, SEQ ID N°2 ou SEQ ID N°3 ou d'une séquence peptidique homologue.

Ces séquences SP permettent de détecter la périostine totale sans distinction des différentes isoformes de cette protéine. Ces séquences proviennent de la partie constante de la périostine. Ce sont des séquences peptidiques courtes, très exposées et hautement conservées entre les différentes espèces, en particulier entre l'homme et la souris. Ces séquences de détection données et leurs homologues permettent de détecter de façon précoce et de façon fiable des phénomènes biologiques ou des pathologies impliquant la périostine et ceci sans être limité à une isoforme particulière de la périostine.

Ces nouvelles séquences de détection spécifiques de la périostine et leurs homologues comprennent tout ou partie des trois séquences originelles annexées SEQ ID N°1, SEQ ID N°2 et SEQ ID N°3.

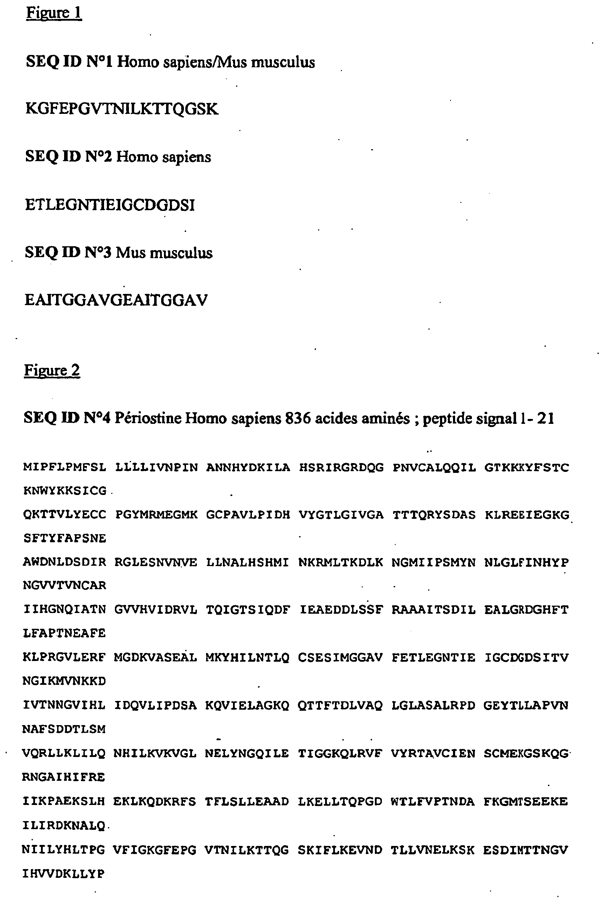

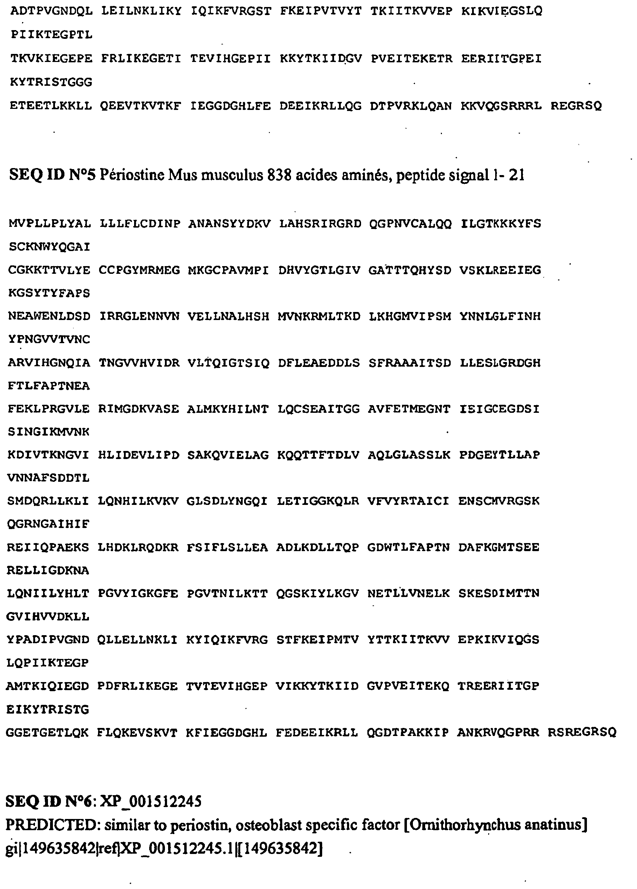

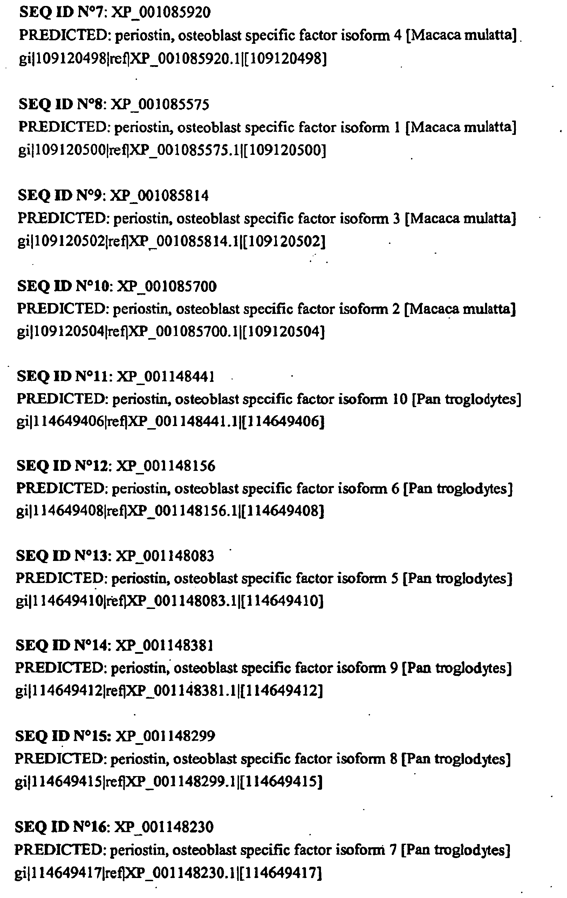

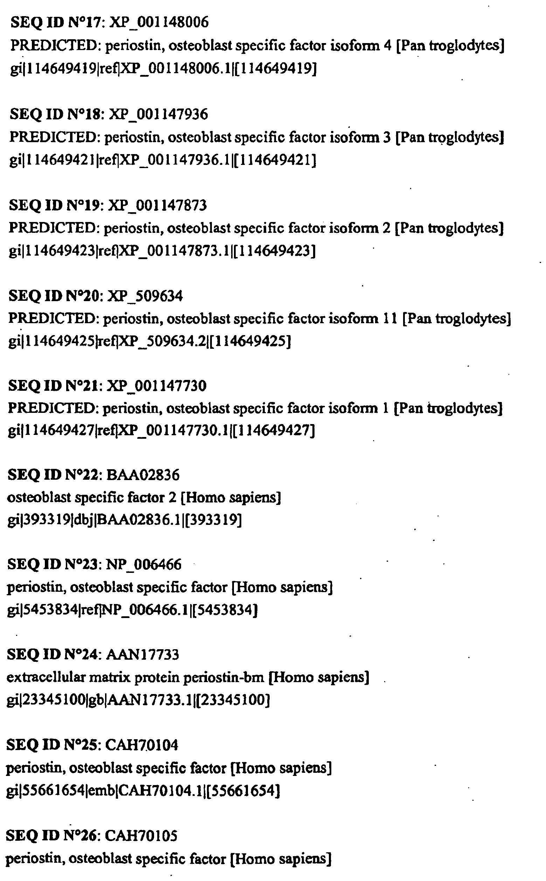

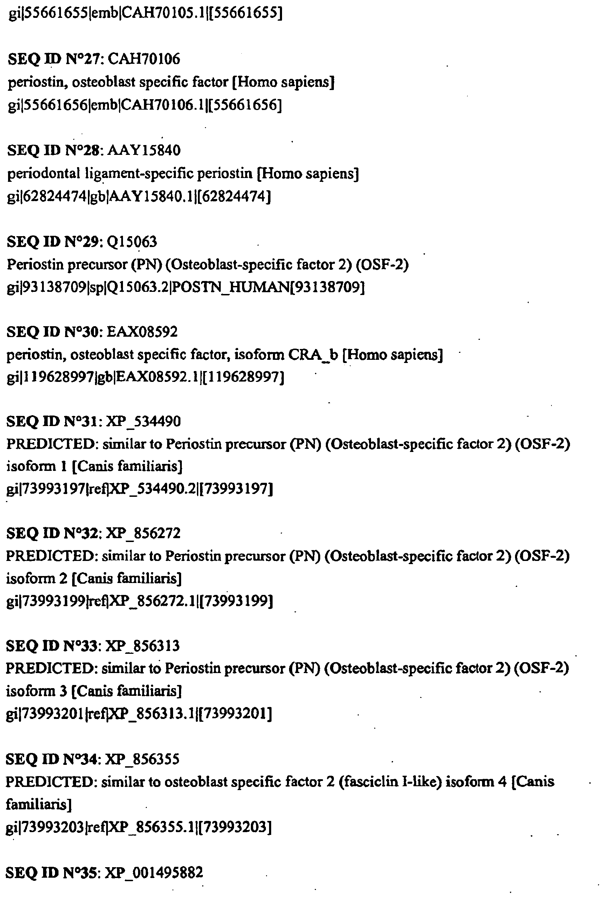

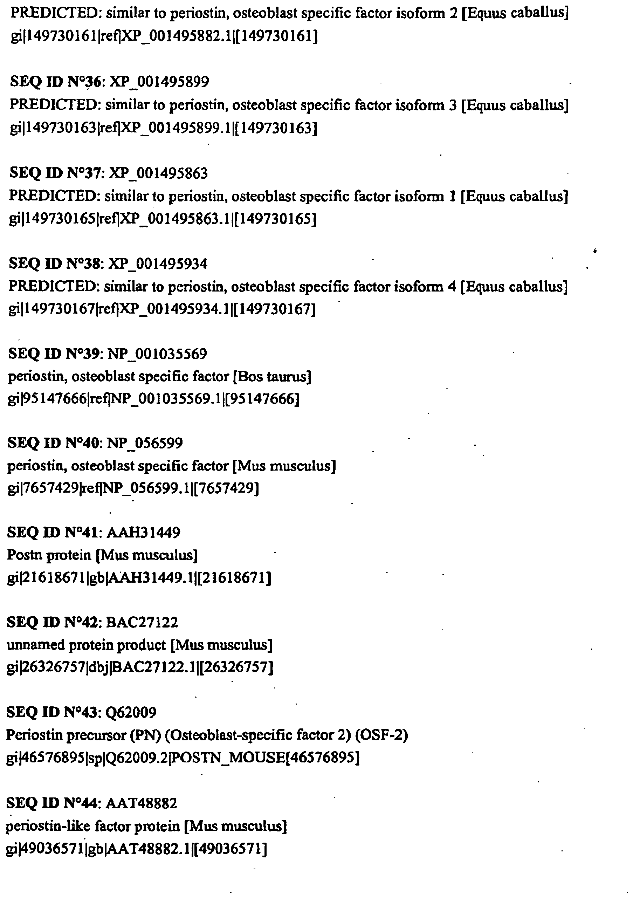

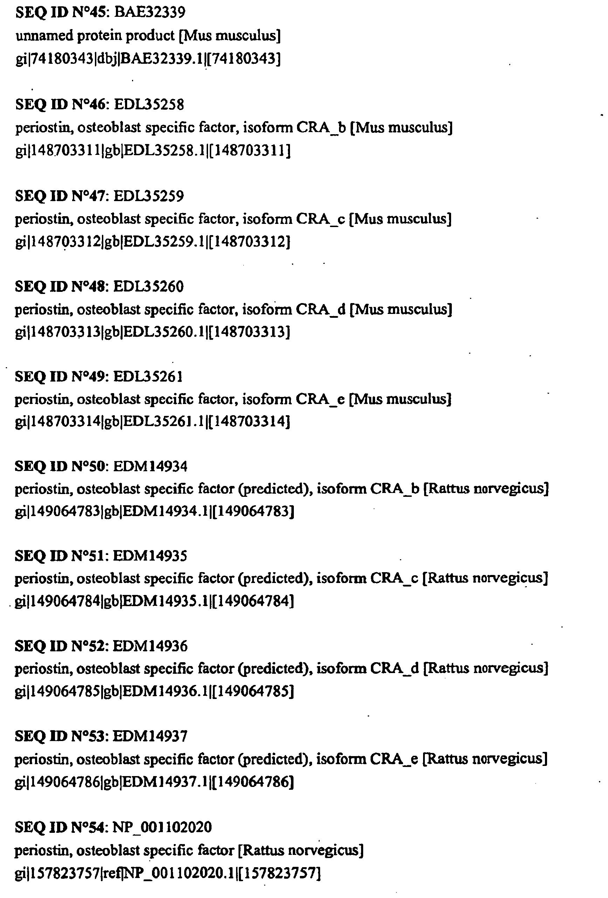

SEQ ID N°1 se retrouve intégralement dans la périostine humaine, du rat, de la souris, du singe, etc, comme cela ressort des séquences peptidiques annexées SEQ ID N°4 à SEQ ID N°54 (figure 2).

SEQ ID N°2 est spécifique de la périostine humaine (SEQ ID N°4).

SEQ ID N°3 est spécifique de la périostine murine (SEQ ID N°5).

| **Numéro de séquence SEQ ID** | **Référence de la séquence de la protéine dans la base de données NCBI http://www.ncbi.nim.nih.gov/protein/** |
|---|---|
| SEQ ID N°6 | XP_001512245 |
| SEQ ID N°7 | XP_001085920 |
| SEQ ID N°8 | XP_001085575 |
| SEQ ID N°9 | XP_001085814 |
| SEQ ID N°10 | XP_001085700 |
| SEQ ID N°11 | XP_001148441 |
| SEQ ID N°12 | XP_001148156 |
| SEQ ID N°13 | XP_001148083 |
| SEQ ID N°14 | XP_001148381 |
| SEQ ID N°15 | XP_001148299 |
| SEQ ID N°16 | XP_001148230 |
| SEQ ID N°17 | XP_001148006 |
| SEQ ID N°18 | XP_001147936 |
| SEQ ID N°19 | XP_001147873 |
| SEQ ID N°20 | XP_509634 |
| SEQ ID N°21 | XP_001147730 |
| SEQ ID N°22 | BAA02836 |
| SEQ ID N°23 | NP_006466 |
| SEQ ID N°24 | AAN17733 |
| SEQ ID N°25 | CAH70104 |
| SEQ ID N°26 | CAH70105 |
| SEQ ID N°27 | CAH70106 |
| SEQ ID N°28 | AAY15840 |
| SEQ ID N°29 | Q15063 |
| SEQ ID N°30 | EAX08592 |
| SEQ ID N°31 | XP_534490 |
| SEQ ID N°32 | XP_856272 |
| SEQ ID N°33 | XP_856313 |
| SEQ ID N°34 | XP_856355 |
| SEQ ID N°35 | XP_001495882 |
| SEQ ID N°36 | XP_001495899 |
| SEQ ID N°37 | XP_001495863 |
| SEQ ID N°38 | XP_001495934 |
| SEQ ID N°39 | NP_001035569 |
| SEQ ID N°40 | NP_056599 |
| SEQ ID N°41 | AAH31449 |
| SEQ ID N°42 | BAC27122 |
| SEQ ID N°43 | Q62009 |
| SEQ ID N°44 | AAT48882 |
| SEQ ID N°45 | BAE32339 |
| SEQ ID N°46 | EDL35258 |
| SEQ ID N°47 | EDL35259 |
| SEQ ID N°48 | EDL35260 |
| SEQ ID N°49 | EDL35261 |
| SEQ ID N°50 | EDM14934 |
| SEQ ID N°51 | EDM14935 |
| SEQ ID N°52 | EDM14936 |
| SEO ID N°53 | EDM14937 |
| SEQ ID N°54 | NP_001102020 |

Au sens de l'invention, on désigne e.g. par "séquence homologue", on entend une séquence ayant quelques variations par rapport à la séquence originelle, ici SEQ ID N°1, SEQ ID N°2 ou SEQ ID N°3. Ces variations viennent du fait que dans les protéines il peut y avoir des délétions, des additions, des substitutions et/ou des insertions de un plusieurs acides aminés mais sans que celles-ci n'affectent de façon majeure la structure et la fonction de la protéine intégrant ladite séquence, en l'occurrence la périostine. Cette dernière conserve ainsi par exemple son activité (telle que l'activité enzymatique) et/ou son repliement tridimensionnel. Selon la présente invention, les substitutions sont définies *e.g.* en tant qu'échanges dans un des groupes suivants :
- petit résidu aliphatique, non polaire ou faiblement polaire : Ala, Ser, Thr, Pro, Gly
- résidu chargé négativement, polaire et leur amide : Asp, Asn, Glu, Gln
- résidu chargé positivement, polaire : His, Arg, Lys
- grand résidu aliphatique, non polaire : Met, Leu, Ile, Val, Cys
- grand résidu aromatique : Phe, Tyr, Trp.

Ainsi les changements qui résultent d'une substitution d'un résidu chargé négativement par un autre (tel que l'acide glutamique par l'acide aspartique) ou un résidu chargé positivement par un autre (tel que Lysine par Arginine) ou des modifications post-traductionnnelles comme glysosylation, nitration, isomérisation...peuvent donner des produits équivalents au plan fonctionnel et tridimensionnel.

Au sens de l'invention, une séquence est dite "homologue" de SEQ ID N°1, SEQ ID N°2 ou SEQ ID N°3, si son degré d'homologie avec SEQ ID N°1, SEQ ID N°2 ou SEQ ID N°3, est, selon un index croissant de préférence, supérieur ou égal à 80%, 85%, 90%, 95%, et 97%.

Les positions sur lesquelles les acides aminés sont modifiés et le nombre d'acides aminés sujets à une modification dans la séquence d'acide aminé SEQ ID N°1, SEQ ID N°2 ou SEQ ID N°3 n'est pas limité. L'homme de l'art est capable de reconnaître les modifications qui peuvent être introduites sans affecter l'activité et/ou le repliement de la protéine. Par exemple, une modification dans la région N ou C terminale d'une protéine est supposée ne pas altérer l'activité fonctionnelle ou le repliement de la protéine dans certaines circonstances.

Dans une première forme préférée de réalisation, SP correspond à SEQ ID N°1.

Cette séquence SEQ ID N°1 est une séquence peptidique de la périostine hautement conservée. En effet, SEQ ID N° (dont la séquence est KGFEPGVTNILKTTQGSK) est conservée avec 100% d'homologie entre la souris, l'homme et de nombreuses autres espèces telles que le singe, le chien, le cheval, le rat (figure 2).

Dans une deuxième forme préférée de réalisation, SP correspond à SEQ ID N°2.

Dans une troisième forme préférée de réalisation, SP correspond à SEQ ID N°3.

Ces séquences SEQ ID N°2 et SEQ ID N°3 sont des séquences peptidiques de la périostine hautement conservées. SED ID N°2 (dont la séquence est ETLEGNTIEIGCDGDSI) est spécifique de la périostine humaine (SEQ ID N°4) et SEQ ID N°3 (dont la séquence est EAITGGAVGEAITGGAV) est spécifique de la périostine murine (SEQ ID N°5) qui contient la séquence monomérique EAITGGAV.

La (ou les) séquence(s) peptidique(s) de détection SP selon la présente divulgation est un peptide qui peut exister en tant que tel ou être inclus dans un (poly)peptide plus long.

Ainsi, au sens de la présente divulgation, l'expression *"séquence(s) peptidique(s) de détection SP"* englobe également tout (poly)peptide immunogène incluant SP telle que définie dans la présente demande et comportant plus de 30 acides aminés.

Comme cela sera vu infra, aussi bien SP que tout (poly)peptide immunogène incluant SP permet la production d'anticorps spécifiques, en particulier dirigés contre tout ou partie de la séquence peptidique SEQ ID N°1, SEQ ID N°2 ou SEQ ID N°3.

Selon un mode particulier de réalisation, la présente invention concerne un antigène de séquence peptidique SP consistant en 6 à 30 acides aminés (de préférence 15 à 26) et incluant la séquence peptidique SEQ ID N°1, SEQ ID N°2 ou SEQ ID N°3 ou une séquence peptidique homologue.

Avantageusement, SP est isolée de son environnement naturel ou produite à l'aide d'un procédé technique.

Par "isolée", on entend e.g. au sens du présent exposé, une séquence peptidique ou une protéine qui a été séparée ou purifiée des composés qui l'accompagne dans son milieu naturel, par exemple dans son tissu d'origine tel que l'os, le coeur, un tissu tumoral ou d'un liquide corporel tel que le sang, la lymphe, l'urine, la salive. Selon la présente invention, la séquence peptidique SP en question est de préférence isolée de l'os et plus préférentiellement du périoste.

Une séquence peptidique isolée selon l'invention peut être obtenue, par exemple, par extraction de sa source naturelle (tel que des tissus ou des liquides corporels) ; par expression par un acide nucléique recombinant codant pour ladite séquence peptidique ; ou par synthèse chimique. Le degré de séparation/purification de ladite séquence peptidique peut être vérifié par différentes méthodes connues de l'homme de l'art telles que la chromatographie sur colonne, des analyses en chromatographie en phase liquide à haute performance (HPLC) ou par électrophorèse sur gel de polyacrylamide.

SP peut être également produite à l'aide d'un procédé technique connu, e.g. : synthèse peptidique, synthèse génétique, ou technique du type de celle décrite dans "Ed Harlow and David Lane « Antibodies : a laboratory manual » Cold Spring Harbor Laboratory press 1988".

### Séquences nucléiques codant pour les séquences peptidiques SP de détection

L'invention concerne également les outils génétiques de synthèse des séquences peptidiques SP, en particulier des séquences SEQ ID N°1, SEQ ID N°2 ou SEQ ID N°3, ou de leurs séquences homologues.

Ces outils génétiques de synthèse sont les séquences nucléiques codant au moins l'une des séquences SP de détection de la périostine telles que définies ci-dessus, y compris les *"(poly)peptides immunogènes"* susvisés.

Les séquences nucléiques en question peuvent être des séquences d'ADNc, d'ADN génomiques ou synthétiques ou des séquences d'ARN. Elles peuvent être à simple brin ou à double brin. Ces séquences peuvent être produites par PCR ou à l'aide d'enzymes de restriction. Ces séquences peuvent contenir également des séquences qui diffèrent de celles générées naturellement mais qui, du fait de la dégénérescence du code génétique, permettent tout de même de coder la même séquence protéique et en particulier une séquence contenant SEQ ID N°1, SEQ ID N°2 ou SEQ ID N°3, ou une séquence homologue à l'une des susdites séquences avec un degré d'homologie tel que défini ci-dessus. Ces séquences nucléiques ne sont bien évidemment pas limitées à des séquences codantes uniquement, elles peuvent contenir des séquences non-codantes.

On aura compris que grâce aux séquences nucléiques ci-dessus décrites, par des procédés d'isolation ou de production protéique connus, on obtiendra les séquences peptidiques isolées de leur environnement naturel ou produites artificiellement telles que décrites ci-dessus et que ces séquences peptidiques correspondent à des peptides immunogènes capables d'engendrer la formation d'anticorps spécifiques.

### Moyens de détection de la périostine : anticorps

Conformément à l'invention, les séquences peptidiques SP de détection sont d'excellentes cibles spécifiques de la périostine et repérables dans un procédé d'analyse qualitative et/ou quantitative de la périostine.

Il convient donc de prévoir, dans un tel procédé, des sondes capables de reconnaître lesdites cibles. C'est là un autre objet de la présente invention, à savoir des moyens de détection de la périostine, qui comprennent un outil de reconnaissance spécifique d'une ou plusieurs séquences peptidiques SP tel que définies à la revendication 1. Cet outil comporte au moins un anticorps anti-périostine et/ou au moins l'un de ses fragments fonctionnels (Ac anti-périostine), spécifiques de SEQ ID N°1, SEQ ID N°2, SEQ ID N°3 ou d'une séquence peptidique présentant un degré d'homologie supérieur ou égal à 80%, de préférence supérieur ou égal à 85% vis-à-vis d'au moins l'une des séquences SEQ ID N°1, SEQ ID N°2 et SEQ ID N°3.

L'outil de reconnaissance spécifique de la périostine est de préférence un anticorps anti-périostine ou au moins l'un des fragments fonctionnels de celui-ci. Dans une forme tout particulièrement préférée de réalisation, l'anticorps selon l'invention est dirigé contre au moins l'une des séquences: SEQ ID N°1, SEQ ID N°2 ou SEQ ID N°3.

Dans la présente demande, le terme "anticorps" désigne e.g. des immunoglobulines (Ig), par exemple de type Gamma (Ig G) ou Mu (Ig M), de préférence IgG. Ces anticorps peuvent être polyclonaux ou monoclonaux. Ils sont issus d'immunisation d'animaux tels que le lapin (polyclonaux), la souris (monoclonaux) et tous les animaux que l'on peut immuniser et connus de l'homme de l'art mais ils peuvent être produits par génie génétique. Par ailleurs, les anticorps selon l'invention peuvent être des anticorps d'origine animale (de préférence du lapin ou de la souris) naturels ou humanisés. Par extension, les anticorps selon la présente invention peuvent être un des fragments fonctionnels desdits anticorps comme le fragment Fv, le fragment Fab qui contient le site de reconnaissance spécifique à l'antigène, le fragment F(ab')₂ qui contient au moins les deux sites de reconnaissance spécifique à l'antigène. Il peut également s'agir d'anticorps chimères.

Dans la présente demande, le terme "épitope" désigne e.g. une molécule ou une région de celle-ci qui peut être reconnue par la partie de reconnaissance spécifique d'un anticorps (le paratope). Un antigène est caractérisé par ses épitopes.

Ainsi, conformément à l'invention, les antigènes aptes à être reconnus et se complexer avec les moyens de détections de la périostine, sont les séquences SP telles que définies dans le présent exposé. En outre, les épitopes correspondant sont notamment les séquences peptidiques SP de 6 à 30 acides aminés correspondant en tout ou partie à SEQ ID N°1, SEQ ID N°2 ou SEQ ID N°3 ou à une séquence homologue à l'une de celles-ci. Il est connu de l'homme de l'art qu'un site antigénique contient généralement 5 à 20 acides aminés mais selon un mode préféré de l'invention, l'épitope antigénique spécifique reconnu par l'anticorps peut contenir un nombre inférieur d'acides aminés, c'est-à-dire de 6 acides aminés à 16 acides aminés sans se limiter à ce nombre. L'épitope contient de préférence 12 acides aminés.

Par "spécifique", on entend e.g. au sens du présent exposé que les anticorps reconnaissent et se lient à un épitope donné via leur paratope selon les principes connus de l'immunologie. En particulier, l'antigène reconnu est défini par son épitope particulier et ceci selon la structure ou la conformation de celui-ci.

Ainsi, les anticorps selon la présente invention permettent de reconnaître spécifiquement toutes les isoformes de la périostine contenant SEQ ID N°1, SEQ ID N°2 ou SEQ ID N°3.

### Procédé de production de moyens de détection (anticorps) de la périostine

Ce procédé selon la description comprend les étapes abcde(f) définies ci-dessus et détaillées ci-après.

### Etape a:

Plus précisément et selon une 1^{ère} modalité avantageuse de la description permettant de générer ces anticorps spécifiques de la périostine via l'épitope de séquence SEQ ID N°1 ou une séquence homologue à celle-ci telle que décrite ci-dessus, il est prévu d'utiliser par exemple une séquence peptidique dérivée des séquences de périostine humaine SEQ ID N°4, murine SEQ ID N°5 et toute autre séquence citée SEQ ID N°6 à SEQ ID N°54 (fig.2) produite artificiellement et comprenant au moins SEQ ID N° 1 ou une séquence homologue à celle-ci. Cette séquence peptidique isolée ou produite artificiellement mime la région identique ou homologue de la périostine native ou d'une isoforme.

Plus précisément et selon une 2^{ème} modalité avantageuse de la description permettant de générer ces anticorps spécifiques de la périostine via l'épitope de séquence SEQ ID N°2 ou une séquence homologue à celle-ci telle que décrite ci-dessus, il est prévu d'utiliser par exemple une séquence peptidique dérivée des séquences de périostine humaine SEQ ID N°4, SEQ ID N°22 à SEQ ID N°30.

Plus précisément et selon une 3^{ème} modalité avantageuse de la description permettant de générer ces anticorps spécifiques de la périostine via l'épitope de séquence SEQ ID N°3 ou une séquence homologue à celle-ci telle que décrite ci-dessus, il est prévu d'utiliser par exemple une séquence peptidique dérivée des séquences de périostine murine SEQ ID N°5, SEQ ID N°40 à SEQ ID N°49.

### Etape b:

Une fois l'antigène produit ou isolé (étape a), il est couplé à une protéine porteuse. Par le terme « couplé », on entend par exemple lié ou conjugué. Cette liaison ou conjugaison peut se faire chimiquement, par exemple par une liaison covalente, adsorption ou un autre mode de fixation connu de l'homme de l'art. De préférence, le couplage se fait de préférence par un agent de couplage comme par exemple le carbodiimide ou la glutaraldéhyde.

La molécule porteuse à laquelle est couplé l'antigène est une molécule de poids moléculaire suffisamment important pour induire une réaction immunitaire et la production d'anticorps circulants. Cette molécule peut être une protéine, un polymère naturel ou synthétique. Elle est de préférence une protéine et en particulier une protéine de poids moléculaire supérieur ou égal à 5 kDaltons telle que la BSA (Bovine Sérum Albumin), l'ovalbumine, la KLH (Keyhole Limpet Hemocyanine). Dans une forme préférée de l'invention, on utilise la protéine KLH en tant que protéine porteuse.

### Etape c:

Le couple antigène-molécule porteuse est injecté à un animal à immuniser. Le couple antigène-molécule porteuse est de préférence en suspension et de préférence mélangé avec une solution d'adjuvant de Freund qui est composée d'huile minérale additionnée d'un agent émulsifiant (adjuvant incomplet) et de particules de bacille inactivé de la tuberculose (adjuvant complet).

L'animal auquel est injecté ledit couple antigène-molécule porteuse en suspension peut être le lapin, la souris, le rat, la chèvre, le mouton, le cheval ou le cobaye, de préférence le lapin, le rat et la souris. L'injection dudit couple peut se faire en une seule fois ou en plusieurs fois selon les méthodes classiques et connues d'immunisation. Les principales voies d'administration sont des injections sous-cutanées, intradermiques, ou intramusculaires; les injections intraveineuses et intra-péritonéales ne sont employées que dans des cas particuliers. S'il y a plusieurs injections du couple antigène-molécule porteuse, les types d'anticorps produits, spécifiques de l'antigène tel que défini ci-dessus, sont différents. En effet, il peut s'agir d'immunoglobulines de type G ou M. Les méthodes d'injection sont connues et sont des méthodes classiques dans les protocoles d'immunisation d'animaux, particulièrement de lapins. Ainsi, on injecte de préférence en intramusculaire environ 0,5 à 1 mg dudit couple antigénique à un lapin et environ 50 à 100 µg à une souris.

Le sang de l'animal contenant, entre autres, lesdits anticorps spécifiques de l'antigène injecté est récupéré par prélèvement ou par exsanguination. De préférence, on prélève du plasma ou du sérum contenant également, entre autres, les anticorps spécifiques de l'antigène tel que défini ci-dessus. Le sérum contenant les anticorps recherchés, dirigés contre ledit antigène, contient normalement plusieurs espèces d'anticorps différents dirigés contre plusieurs épitopes du même antigène, on appelle cette préparation polyclonale. On peut également mettre en oeuvre des techniques permettant d'isoler et de cloner des lymphocytes ne produisant qu'une espèce moléculaire (i.e. clonotype) d'anticorps : les anticorps qui ne reconnaissent qu'un seul épitope de l'antigène.

### Etape d:

L'étape de sélection (ou titration) des anticorps spécifiques dudit antigène tel que défini ci-dessus se fait à l'aide de la technique ELISA (Enzyme-Linked Immuno Sorbent Assay). Cette technique de sélection est largement décrite dans la littérature.

### Etape e:

Lorsque cette étape facultative de purification des anticorps est réalisée, cette dernière peut être faite en phase liquide ou solide et de différentes façons à savoir la chromatographie d'affinité en utilisant soit des colonnes de séparation, des billes magnétiques ou des résines portant ledit antigène en tant que ligand. On préfère un support solide tel qu'une colonne de sépharose sur laquelle les antigènes ont été fixés, de préférence par liaison covalente. On fait passer le sang/sang/plasma/sérum sur ladite colonne de fixation où seuls les anticorps contre l'antigène devraient s'attacher sur la colonne, toutes les autres protéines du sérum étant lavées dans l'éluat permettant la sélection des anticorps spécifiques des antigènes.

### Procédé de détection et éventuellement de dosage de la périostine totale dans un échantillon biologique

La présente invention donne accès aux séquences de détection SP, lesquelles permettent de produire des moyens de détection constitués e.g. par un outil reconnaissance de la périostine, de préférence les anticorps anti-périostine. Cet outil spécifique rend possible la détection in vitro (analyse quantitative) voire le dosage (analyse quantitative) de la périostine totale porteuse de tout ou partie de SP, par le procédé défini ci-avant et comportant les étapes I-II-III-(IV).

Les séquences SP de détection de la périostine telles que définies dans le présent exposé sont particulièrement avantageuses car elles permettent de distinguer, quantifier et/ou localiser la périostine exprimée dans l'organisme et ceci sans distinction entre ses différentes isoformes (5) jusqu'à présent répertoriées (fig. 2), ni des modifications post traductionnelles telles que la carboxylation, la glycosylation...

La détection et/ou la quantification (concentration) du marqueur protéique qu'est la périostine, via les séquences de détection SP selon l'invention, dans un échantillon biologique d'un sujet (animal ou humain) est particulièrement intéressante car elle renseigne de façon précoce et fiable sur les phénomènes biologiques non pathologiques se produisant dans certains tissus chez cet individu, en particulier l'os mais également sur d'éventuelles pathologies impliquant la périostine. Elle constitue ainsi un indice majeur soit de l'état physiologique d'un sujet au plan du métabolisme osseux, en particulier du périoste et/ou du cartilage, notamment lors de la croissance ou lors de l'andropause ou la ménopause, soit de l'état pathologique de l'individu atteint ou susceptible d'être atteint par des maladies impliquant la périostine, notamment les pathologies osseuses telles que l'ostéoporose, les métastases osseuses du cancer du sein et de la prostate et d'autres origines, le myélome, ou les pathologies ostéoarticulaires telles que l'arthrose, la polyarthrite rhumatoïde, la spondylarthrite, les fibroses notamment hépatique, et les maladies cardiovasculaires.

L'invention apporte donc à un progrès technique significatif au travers de cet outil spécifique, fiable et facile d'utilisation de reconnaissance et de détection de ce marqueur périostine, lequel outil constituant le fondement du procédé dont il est question dans cette rubrique et sur les étapes duquel on donne quelques précisions ci-après.

Dans ce cadre, la détection du complexe antigène-anticorps peut être déclinée selon plusieurs technologies telles que les tests ELISA, EIA ou RIA, l'immunoturbidimétrie, l'agglutination de latex sur lame, la néphélométrie, la turbidimétrie, la turbidimétrie ou néphélométrie amplifiée par particules de latex, l'immunohistochimie/cytochimie colorimétrique, l'immunofluorescence (sur lame), microplaque, latex fluorescent, latex magnétique, test sur membrane, biochips etc...), les buvardages en western (western blot), les transferts en point (dot blot), les techniques de chromatographie liquide à haute performance (HPLC), par électrophorèse, par spectroscopie, ou par les techniques d'analyse protéomique ou "microarray", et de façon générale peut être utilisé tout procédé permettant d'identifier et/ou de quantifier une réaction entre un antigène et plus particulièrement un épitope et un anticorps, quelque soit l'isotype. De préférence, on utilise un test ELISA. Dans une forme particulière de réalisation de l'invention, ledit procédé de détection de la périostine consiste en un dosage ELISA, de préférence un dosage ELISA dit « par compétition » ou dit « sandwich ».

Dans une variante, on peut utiliser des techniques permettant la détection directe des anticorps en tant que complexes anticorps-épitope telle que la réfractométrie, la diffraction de rayons lumineux par la surface réactionnelle, des méthodes de modification de la conductivité électrique ou du champ magnétique etc....

Les méthodes d'immunochimie de détection utilisent généralement une réaction enzymatique (peroxydase, glucose oxydase, phosphatase alcaline). De préférence, la réaction immunologiques, à savoir la reconnaissance de l'anticorps ou de au moins l'un de ses fragments fonctionnels selon l'invention avec l'antigène tel que défini ci-dessus, est suivie par une réaction indicatrice qui produit un signal coloré amplifié par des enzymes et leurs substrats chromogènes ou qui permet la détection photométrique, électrochimique fluorescente ou radioactive etc. de l'activité enzymatique liée à la concentration du complexe anticorps-antigène.

Ces techniques génériques peuvent ainsi s'inscrire dans les étapes I-II-III-(IV) suivantes.

### Etape I:

Au sens de l'invention, on désigne e.g. par le terme "échantillon biologique," tout type d'échantillon, utilisable in vitro, de toute substance susceptible de contenir ledit antigène en question correspondant à une séquence peptidique SP, à savoir :
- une substance liquide tels que le sérum, le plasma, le sang, de la lymphe, de l'urine, de la salive, le surnageant de cellules mises en culture, la moelle osseuse, un broyat d'organe, des extraits cytoplasmiques de cellules données;
- ou une substance solide, par exemple des coupes de tissus (par exemple osseux ou tumoraux), un organe ou une coupe de celui-ci.

Ces échantillons sont prélevés sur des animaux ou des patients sains ou atteints de maladies, par exemple le cancer du sein ou de la prostate.

De préférence, on utilise du sérum.

Les anticorps peuvent être (technique sandwich) ou pas (technique de compétition) préalablement fixés sur support adapté.

Les supports de réactions immunologiques utilisés peuvent être de natures différentes et sont choisis parmi : les lames, les microplaques, les latex fluorescents, les latex magnétiques, les membranes d'immuno-filtration ou les membranes d'immuno-migration, les biochips, les billes, les ailettes, les tubes mais également les liposomes, les vésicules lipidiques, les microparticules biologiques ou obtenues à partir de polymères, les émulsions ou tout autre support connu de l'homme de l'art et adapté à la mise en oeuvre desdits procédés.

Les éventuels lavages servent à éliminer les fixations non spécifiques ou les produits en excès non fixés. Selon des méthodes connues de l'homme de l'art, l'anticorps spécifique peut être utilisé en phase liquide, en solution. De préférence, l'outil de reconnaissance de la séquence de détection (anticorps ou l'un de leurs fragments fonctionnels) selon la présente invention est, au préalable, fixés sur un support essentiellement solide ou tout support réactionnel permettant de réaliser un test immunologique. La fixation au support est réalisée, de préférence, par adsorption ou liaison covalente. Une éventuelle étape de lavage permet de supprimer après ladite fixation les anticorps non fixés audit support le cas échéant.

### Etapes II-III-(IV):

Lorsque l'échantillon contient des molécules contenant au moins cet antigène, des molécules de périostine, ces dernières via l'épitope vont se lier spécifiquement aux anticorps pouvant être (technique sandwich) ou pas (technique de compétition) préalablement fixés sur support adapté.

Ainsi, la détection de la périostine permet de déterminer le niveau d'expression de cette protéine et, compte tenu du fait que l'anticorps est dirigé contre un épitope très exposé dans la partie commune de la périostine, ce procédé de détection permet de déterminer le niveau d'expression de la périostine totale, toutes formes confondues de la protéine. La mise en oeuvre de ce procédé de détection, grâce aux séquences de détection SP selon l'invention et l'anticorps tels que définis ci-dessus, est particulièrement intéressant car il va permettre de mieux comprendre ou définir les rôles de la périostine dans certains processus biologiques car cette protéine de la matrice extracellulaire du périoste est impliquée dans différents métabolismes et phénomènes biologiques, pathologiques ou non encore mal compris. Ainsi, ce procédé de détection permet de mieux définir, comprendre et déterminer entre autres, l'activité métabolique de l'os au cours de la vie d'un individu donné. Puisque la périostine est fortement exprimée au niveau périostéal et que le périoste est une structure dont le rôle est capital pour la solidité osseuse, il a été envisagé d'utiliser cette protéine comme marqueur de l'activité métabolique du périoste. Ainsi, le niveau d'expression de ce marqueur pourra être un indicateur de l'action d'hormones ou d'agents thérapeutiques sur le périoste. Contrairement à l'implication de la périostine dans les mécanismes du cancer, aucune publication n'a encore fait état de son utilité comme marqueur du remodelage osseux, et plus particulièrement du métabolisme périostéal.

Dans une variante de mise en oeuvre, le procédé de détection de la périostine, dans un échantillon biologique in vitro, est basé sur la technique de dosage ELISA par compétition et comprend les étapes suivantes :
I'. Fixer sur un support approprié un peptide SP antigène synthétique biotinylé comprenant tout ou partie de l'épitope contenu dans SP et en particulier dans SEQ ID N°1, SEQ ID N°2 SEQ ID N°3 et/ou dans une séquence homologue à l'une de celles-ci, le degré d'homologie étant tel que défini ci-dessus,
II'. Mettre en présence ledit support de l'étape l' avec, d'une part, un échantillon biologique susceptible de contenir la périostine et, d'autre part, des anticorps dirigés contre au moins l'un desdits peptides SP tels que décrits ci-dessus ou l'un de leurs fragments fonctionnels,
II'.1. Effectuer au moins un lavage,
III'. Révéler les éventuels complexes Ac anti-périostine/SP formés,
IV'. Doser les éventuels complexes Ac anti-périostine/SP formés par mesure du marquage ou de la densité optique pour en déduire la concentration de la périostine contenue dans l'échantillon. Plus la densité optique est faible, plus l'échantillon biologique contient de périostine.

Dans une autre variante de mise en oeuvre, le procédé de détection de la périostine, dans un échantillon biologique in vitro, est basé sur la technique ELISA dite « sandwich » et comprend les étapes suivantes :
I". éventuellement fixer sur un support approprié les anticorps ou au moins l'un de leurs fragments fonctionnels tels que définis ci-dessus,
II". mettre en présence un échantillon contenant potentiellement ledit antigène correspondant à tout ou partie de SP et en particulier de SEQ ID N°1, SEQ ID N°2 SEQ ID N°3 et/ou d'une séquence homologue à l'une de celles-ci, avec les anticorps ou au moins l'un de leurs fragments fonctionnels tels que définis précédemment,
II".1. effectuer au moins un lavage,
III". détecter/révéler les complexes anticorps-antigènes éventuellement formés.
Procédé de détection et de dosage de la périostine sous-carboxylée (*psc*), à distinguer de la périostine carboxylée (*pc*)

Ce procédé défini supra comprend les étapes I*^{psc}* I*^{psc}*.1 I*^{psc}*.2 I*^{psc}*.3 II*^{psc}* III*^{psc}* IV*^{psc}*

Les séquences de détection SP et les moyens de détection y associés (outil de reconnaissance de SP -de préférence les anticorps-), permettent aussi de détecter et de doser des formes particulières *pc* et *psc* de la périostine. En effet, il est du mérite des inventeurs d'avoir observer que la périostine *pc* carboxylée présente plus d'affinité pour certains supports de fixation et en particulier pour l'hydroxyapatite (de préférence en cristaux) qui entre dans la constitution de la matrice minérale de l'os.

On décrit ci-après un mode préféré de mise en oeuvre.

### Etape I^{psc}.:

I*^{psc}*.1- Tout d'abord l'échantillon biologique contenant la périostine est mis en contact avec des cristaux d'hydroxyapatite.
I*^{psc}*.2- On agite puis on centrifuge.
I*^{psc}.*3- Le surnageant contenant la périostine *psc* est récupéré.
I*^{psc}*. On met en présence ce surnageant avec des moyens de détection selon l'invention : *Ac anti-périostine.*
III*^{psc}*. On met en évidence les éventuels complexes *Ac anti-périostine*/*SP* formés;
*IV^{psc}*. On dose ces complexes *Ac anti-périostine*/*SP* formés pour en déduire la concentration en périostine *psc* dans le surnageant, et *in fine* dans l'échantillon.

Le taux de périostine *pc* est quant à lui déterminé indirectement, c'est-à-dire qu'il correspond à la différence entre le taux de périostine totale (déterminé par dosage de l'échantillon biologique n'ayant pas subi de traitement) et le taux de périostine *psc* (déterminé par dosage de l'échantillon biologique traité à l'hydroxyapatite tel que mentionné ci-dessus).

En d'autres termes, ce procédé de détection et dosage de la périostine *pc* consiste essentiellement à:
1. mettre en oeuvre un échantillon biologique,
2. doser la périostine totale dans cet échantillon biologique,
3. doser la périostine *psc* dans cet échantillon biologique,
4. faire la différence entre la concentration en périostine totale et la concentration en périostine *psc* dans cet échantillon biologique, de façon à obtenir la concentration en périostine *pc* dans cet échantillon biologique.

Comme autres supports de fixation de *pc* ou de *psc* par affinité, on peut citer notamment le sulfate de barium.

De préférence, les procédés de détermination du niveau d'expression de la périostine totale et sous-carboxylée se font directement par ELISA avec les anticorps tels que définis dans la présente demande, dirigés contre tout ou partie de la SEQ ID N°1, SEQ ID N°2 ou SEQ ID N°3 ou l'une de leurs séquences homologues présentant un degré d'homologie vis-à-vis de l'une des susdites séquences tel que défini ci-dessus.

Le taux de carboxylation de la périostine a un impact dans certaines pathologies, par conséquent la détermination de ces deux taux de périostine (carboxylée et sous-carboxylée) peut s'avérer essentielle dans le diagnostic, le suivi ou le pronostic de pathologies.

Les inventeurs ont pu détecter des formes carboxylées de périostine dans le sérum de patients sains. Ils ont également constaté une augmentation des taux sériques de périostine totale chez des patients atteints de cancer de la prostate avec métastases osseuses ainsi qu'une augmentation des taux sériques de périostine carboxylées chez ces mêmes patients en comparaison avec les patients sains.

Il apparaît donc que les procédés et moyens selon la présente invention (séquence de détection et/ou outil de reconnaissance des séquences de détection SP, de préférence les anticorps anti-périostine) sont utilisés afin de déterminer essentiellement :
- le niveau d'expression de la périostine totale (sans distinction d'isoformes ou de modifications post-traductionnelles comme carboxylation, glycosylation, nitration, isomérisation...
- le niveau d'expression respectivement de la périostine décarboxylée et de la périostine carboxylée (indirectement) par rapport au niveau d'expression de la périostine totale,
- l'activité métabolique du périoste,
- le niveau de remodelage osseux et/ou du cartilage articulaire,
   a) lié à l'âge,
   b) lié à un changement hormonal,
   c) lié à l'activité physique
   d) lié à la présence de cellules tumorales,
- le niveau de minéralisation vasculaire,
- la présence ou non de métastases osseuses, de préférence de métastases osseuses du cancer du sein et de la prostate,
- le niveau de réponse stromale aux métastases osseuses du cancer du sein,
- la présence ou non des réactions de fibrose liées aux tumeurs.

Applications du procédé de détection selon l'invention dans l'étude de phénomènes biologiques impliquant la périostine, dans le diagnostic, le suivi ou le pronostic d'une pathologie impliquant la périostine, ou dans la détermination de l'efficacité d'un médicament adapté au traitement d'une pathologie impliquant la périostine

Les moyens et procédés selon l'invention ouvrent de nombreuses portes pour la compréhension et le traitement de pathologies impliquant la périostine, telles que le cancer, les pathologies osseuses telles que l'ostéoporose, les métastases osseuses du cancer du sein et de la prostate et d'autres origines, le myélome, ou les pathologies ostéoarticulaires telles que l'arthrose, la polyarthrite rhumatoïde, la spondylarthrite, les fibroses notamment hépatique, et les maladies cardiovasculaires.

Par ailleurs, en plus de son rôle physiologique dans l'os, de nombreuses études utilisant des modèles in vitro et in vivo ont montré l'implication de la périostine dans différents mécanismes pathologiques tels que le cancer. En effet, on sait que la périostine interagit avec des protéines d'adhésion cellulaires. Elle pourrait avoir un rôle dans l'invasion cellulaire, la survie tumorale, l'angiogenèse et le potentiel métastatique des cellules tumorales. De plus, la périostine joue un rôle dans l'ostéoblastogenèse et pourrait avoir un effet indirect sur l'ostéoblastogenèse, et par conséquent sur l'ostéolyse maligne qui caractérise les métastases osseuses. Ainsi, la détection de la périostine, par le biais d'au moins une nouvelle séquence de détection telle que définie précédemment et au moins un moyen de reconnaissance d'une nouvelle séquence de détection (de préférence un anticorps), est intéressante dans des études cliniques visant non seulement à diagnostiquer, suivre, faire un pronostic ou suivi de traitement, entre autres, des métastases osseuses, des pathologies ostéo-articulaires mais également à constituer une nouvelle approche thérapeutique, entre autres, de l'ostéolyse maligne et de la dégradation du cartilage.

De plus, les inventeurs ont pu montrer que lors d'injections de cellules du cancer du sein humaines à une souris, les cellules stromales murines expriment la périostine en réponse à la présence des cellules tumorales humaines. Ainsi, le dosage de la périostine peut être intéressant pour estimer son rôle potentiel dans la réaction stromale et permettre ainsi la détection très précoce de la périostine lors des processus métastatiques osseux avant la résorption de l'os et donc avant la détection possible par imagerie et avant la détection d'une modification dans le dosage des marqueurs connus du remodelage osseux.

D'où il s'ensuit que l'invention concerne également:
i. un procédé de mise en évidence et d'évaluation d'un phénomène biologique non pathologique impliquant la périostine,
ii. un procédé de diagnostic, de suivi ou de pronostic d'une pathologie impliquant la périostine,
iii. et un procédé de détermination de l'efficacité d'un médicament adapté au traitement d'une pathologie impliquant la périostine, caractérisé en ce qu'il consiste à mettre en oeuvre le procédé selon l'invention tel que décrit supra.

Ces procédés (i), (ii) & (iii) consistent plus précisément et essentiellement :
I. à mettre en oeuvre un échantillon biologique susceptible de contenir de la périostine ou provenant du sujet faisant l'objet d'un diagnostic, d'un suivi, d'un pronostic d'une pathologie impliquant la périostine ou d'une détermination de l'efficacité d'un médicament adapté au traitement d'une pathologie impliquant la périostine,
II. à mettre en présence ledit échantillon biologique avec un outil de reconnaissance spécifique de la périostine tel que défini précédemment, de préférence un anticorps ou l'un des fragments fonctionnels de celui-ci tel que défini ci-dessus,
III. à mettre en évidence la périostine en détectant les éventuels complexes immunologiques *Ac anti-périostine*/*SP* éventuellement formés,
IV. et éventuellement à doser ces complexes *Ac anti-périostine*/*SP* formés pour en déduire la concentration en périostine dans l'échantillon.

Suivant une modalité intéressante de l'invention, il est prévu dans le procédé (i) que le phénomène est choisi dans le groupe de phénomènes comprenant:
l'activité métabolique de l'os, en particulier du périoste, le remodelage de l'os et/ou du cartilage articulaire en fonction de l'âge, du changement hormonal, de l'activité physique, l'ossification intramembranaire, le phénomène de minéralisation vasculaire.

Suivant une autre modalité intéressante de l'invention, il est prévu dans le procédé (ii), qu'au terme de l'analyse quantitative (étapes I-II-III-IV) in vitro de la périostine présente dans un échantillon biologique d'un sujet, on compare la concentration en périostine mesurée dans l'échantillon avec une concentration de référence correspondant à l'existence ou à l'absence de pathologie impliquant la périostine chez un sujet témoin de la même espèce.

De préférence, la concentration de référence correspond soit à une absence de pathologie, soit à l'existence d'une pathologie à un stade déterminé. Ce choix peut être fait par l'homme du métier en fonction du marqueur sélectionné ainsi que de la technique utilisée.

A titre d'exemple, la concentration de référence qui délimite l'état pathologique de l'état non pathologique pour différentes techniques, correspond à la valeur au dessus du 95^{iéme}, de préférence au dessus du 97,5^{ième} percentile des valeurs des sujets sains ou contrôles.

La pathologie considérée dans les procédés (ii) & (iii), est *e.g.* choisie dans le groupe de pathologies comprenant:
- les pathologies osseuses de préférence dans le sous-groupe comprenant l'ostéoporose, les métastases osseuses du cancer du sein et de la prostate et d'autres origines, le myélome, et la dysplasie fibreuse
- et les pathologies ostéoarticulaires de préférence dans le sous-groupe comprenant l'arthrose, la polyarthrite rhumatoïde, la spondylarthrite
- les fibroses notamment hépatiques et les maladies cardiovasculaires.

### Nécessaire de détection et de dosage in vitro de la périostine, dans un échantillon biologique

Ce nécessaire regroupe un certain nombre d'élément utiles pour la mise en oeuvre du procédé susdécrit de détection et de dosage in vitro de la périostine totale, dans un échantillon biologique, à savoir notamment :
→ des moyens de détection de la périostine selon l'invention, qui sont de préférence des *Ac anti-périostine* générés au moyen des séquences SP,
→ des réactifs et du matériel pour la mise en évidence, voire le dosage, des éventuels complexes *Ac anti-périostinelSP* formés, selon les étapes III, voire IV, décrites ci-dessus,
→ et une notice de mise en oeuvre.

L'invention vise également un nécessaire de détection et de dosage de la périostine sous-carboxylée *psc* comprenant:
→ des moyens de détection de la périostine
→ des réactifs et du matériel pour la mise en évidence, voire le dosage, des éventuels complexes *Ac anti-périostine*/*SP* formés selon les étapes III, voire IV décrites ci-dessus,
→ un support pour la mise en oeuvre des étapes I*^{psc}*.et II*^{psc}* décrites ci-dessus, c'est-à-dire un support apte à fixer la *pc* de préférence à la *psc* ou inversement, ledit support étant :
   - avantageusement un support ayant une affinité vis-à-vis *du pc* supérieure à celle vis-à-vis du *psc,*
   - et, de manière plus avantageuse, un support comprenant de l'hydroxyapatite,
   - et, de manière encore plus avantageuse, un support comprenant de la matrice minérale osseuse comprenant de l'hydroxyapatite sous forme de cristaux.
→ et une notice de mise en oeuvre.

### EXEMPLES

Les exemples qui suivent illustrent la présente demande.

L'invention sera mieux comprise si l'on se réfère aux figures annexées dans lesquelles :
- la figure 1 représente les séquences peptidiques des SEQ ID N°1, SEQ ID N°2 et SEQ ID N°3.
- la figure 2 représente les séquences des isoformes (SEQ ID N°4 à SEQ ID N°54) ainsi que les espèces dans lesquelles la séquence SEQ ID N°1 est présente avec 100% d'homologie, ainsi que les séquences humaine SEQ ID N°4 et murine SEQ ID N°5 avec lesquelles les séquences SEQ ID N°2 et SEQ ID N°3 présentent 100% d'homologie respectivement.
- la figure 3 montre des courbes de titration pour la détermination de la concentration en anticorps spécifique du peptide de séquence SEQ ID N°1 en fonction de la quantité de peptide (SEQ ID N°1) biotinylé fixé sur la plaque (figure 3A) et une courbe de titration de l'antisérum pour différentes concentrations du peptide (SEQ ID N°1) adsorbé sur la plaque ELISA (comme indiqué).
- la figure 4 représente la courbe standard de dosage par ELISA du peptide contenant la SEQ ID N°1.
- la figure 5 représente une analyse en western blot de la reconnaissance des périostines recombinantes humaine et murine par l'antisérum spécifique du peptide de séquence SEQ ID N°1.
- la figure 6 est une représentation graphique de dosages de la périostine à l'aide de l'anticorps spécifique de la séquence de détection SEQ ID N°1 selon l'invention, chez des individus sains et des individus atteints de cancer du sein avec ou sans métastases osseuses.
- la figure 7 montre une analyse immunohistochimique de section de tissu osseux de tibia de souris en cours croissance (fig.7A) et adulte (fig.7B) marqué avec l'antisérum spécifique du peptide de séquence SEQ ID N°1.

### Exemple 1 : Procédé de production de l'anticorps anti-périostine selon l'invention.

### 1. Analyse bioinformatique pour identification de la séquence peptidique immunogène. séquence de détection de la périostine.

La sélection bio-informatique se base pour chaque protéine sur:
a - Prédiction d'accessibilité de la séquence de détection :
   - prédiction d'accessibilité de la séquence à un solvant sur la base de la séquence d'acides aminés,
   - prédiction de la structure secondaire,
   - prédiction d'un domaine trans-membranaire
   - une analyse de l'accessibilité en surface, si un modèle en trois dimensions est disponible,
b - Antigénicité : évaluation de la présence de déterminants antigéniques au niveau de la séquence protéique de détection de la périostine :
   - une prédiction de l'antigénicité basée sur les caractéristiques de la séquence d'acides aminés,
   - une analyse des paramètres influençant l'antigénicité (possibilité de mutations, flexibilité, modifications post-traductionnelles)
c - Analyse du potentiel de réactivité croisée :
   - recherche d'homologies intra-espèces
   - recherche de courtes similitudes intra-espèces
d - Application d'un mode de sélection intégrant les précédentes informations pour sélectionner 3 à 5 peptides (environ 17 acides aminés)

### 2. Préparation de la séquence peptidique immunozène, de détection de la périostine contenant au moins SEQ ID N°1 (étape a)

Le peptide de séquence SEQ ID N°1, dérivé de la séquence de la périostine (numéro d'accession dans GenBank : Q15063) a été produit par la technique de synthèse peptidique sur phase solide en utilisant un blocage réversible de l'amine de l'acide aminé par le Fmoc (9-fluorénylméthyloxycarbonyl). La pureté du peptide a été vérifiée par HPLC et par spectrométrie de masse.

### 3. Couplage de la séquence peptidique immunogène de détection de la périostine à une protéine porteuse (étape b)

Le peptide servant d'immunogène, synthétisé avec une cystéine supplémentaire du coté C-terminal, est couplé du côté C-terminal à une protéine porteuse qui est la KLH (keyhole limpet hemocyanine) ce qui va permettre une meilleure réponse immunologique vis-à-vis de ce peptide. Le peptide synthétique qui est adsorbé sur les microplaques a été couplé à la biotine (Harlow et Lane, 1988).

### 4. Injection dudit couple antigène-molécule porteuse à un animal et récupération du sang/sérum (étape c)

2 lapins ont reçu 4 injections par voies intra-péritonéale et sous-cutanée aux jours J0, J14, J28 et J58 de 1ml d'un mélange (50%/50%) d'une solution contenant 200µg du peptide conjugué à la KLH et de l'adjuvant de Freund selon le protocole d'immunisation dans le tableau ci-dessous (étape c). Des prélèvements réguliers (à J0, J36, J70 et J90) de sang ont été effectués pour contrôler la réponse immunitaire (étape d et e). Ce sang est laissé à coaguler pendant 30 minutes puis est centrifugé pendant 10 minutes à 2500 tours par min afin de récupérer le sérum et tester l'immuno-réactivité des anticorps.

| **Jour** | **Protocole** |
|---|---|
| 0 | **T0 : Prélèvement de sérum contrôle (4 - 5 ml)** et conservation à +4°C |
| 0 | **Injection sous-cutanée** (1 ml / lapin) 0,5 ml Antigène + 0,5 ml Adjuvant Freund Complet |
| 14 | **Injection sous-cutanée** (1 ml / lapin) 0,5 ml Antigène + 0,5 ml Adjuvant Freund Incomplet |
| 28 | **Injection sous-cutanée** (1 ml / lapin) 0,5 ml Antigène + 0,5 ml Adjuvant Freund Incomplet |
| 36 | **T1 : Prélèvement de sérum test (4-5 ml)** et conservation à +4°C |
| 58 | **Injection sous-cutanée** (1 ml / lapin) 0,5 ml Antigène + 0,5 ml Adjuvant Freund Incomplet |
| 70 | **T2 : Prélèvement de sérum test (15 ml)** et conservation à +4°C |
| 90 | **T3** : **Prélèvement final** et conservation à +4°C |

### 5. Sélection des anticorps spécifiques de l'pitope dans les sérums : Titration des antisérums (étape d)

Les anticorps anti-périostine spécifiques sont sélectionnés dans le sérum avec l'antigène synthétique de séquence peptidique comprenant au moins une séquence de 6 à 30 acides aminés contenant au moins la séquence SEQ ID N°1 produits ci-dessus grâce à la détermination de l'immunoréactivité du sérum de lapin par la technique ELISA. L'antisérum avec le meilleur titre a été sélectionné pour la mise au point de l'ELISA de compétition (Harlow et Lane, 1988).

Les titres des antisérums ont été réalisés en adsorbant, 2h à température ambiante, le peptide antigénique conjugué à la biotine dilué avec du tampon de revêtement ("coating") sur des plaques revêtues de streptavidine (Nunc, Danemark). Le peptide antigénique biotinylé est dilué à 0 ; 2 ; 5 ; 10 ; 15 et 50 ng/ml. Après lavage, 50 µl d'antisérums dilué du 1/100 au 1/1.000.000 sont ajoutés à 50 µl de tampon de dilution dans les puits et sont mis à incuber pendant 2h à température ambiante. Après lavage, 100 µl d'anticorps secondaire, couplé à la peroxydase, dirigé contre les immunoglobulines de lapins sont ajoutés pendant une heure à température ambiante. Les puits sont lavés et 100 µl de substrat (TMB) sont ajoutés. La réaction est stoppée avec 100 µl d'H₂SO₄.

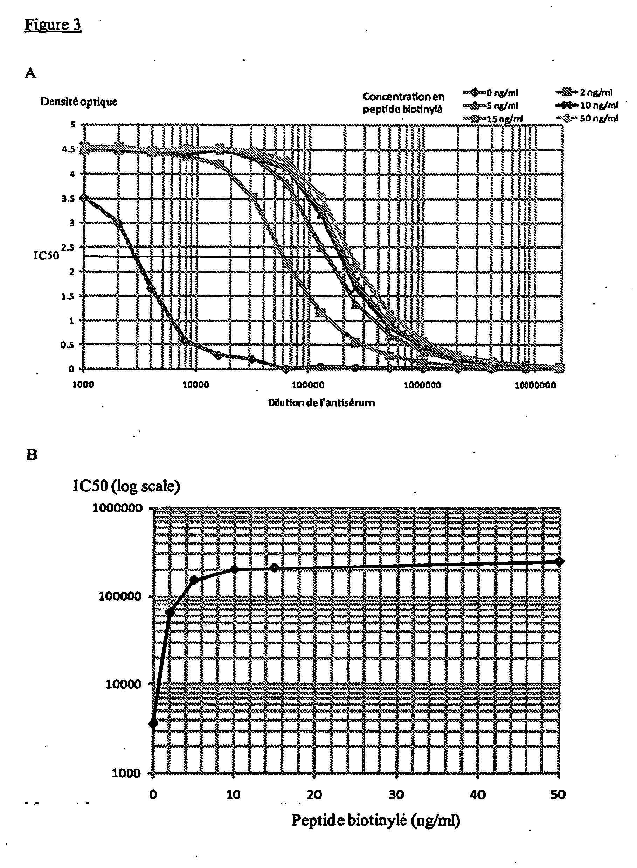

La figure 3A illustre cette titration de l'antisérum contenant l'anticorps dirigé contre le peptide de détection de la périostine contenant SEQ ID N°1. On lit en abscisses les différentes dilutions de l'antisérum (de 0 à 1/16384000) et en ordonnées la densité optique à 450 nm.

La figure 3B illustre la relation entre la concentration en peptide biotinylé coaté et la dilution en anticorps. Le IC 50 est la dilution de l'anticorps correspondant à 50% de la densité optique maximale obtenue avec la plus forte concentration d'anticorps donc la plus petite dilution. Le « coating » à 10 ng/ml sera choisi car on atteint une phase plateau à cette concentration. L'IC50 pour un coating à 10 ng/ml est de 197800.

### 6. Purification des anticorps (étape e)

Les étapes d'immuno-purification des anticorps sont réalisées suivant les protocoles décrits dans la publication Thomas V et al dans J Immunol Methods. 2004 Sep;292 (1-2):83-95.

### Exemple 2 - ELISA de compétition pour la périostine via la séquence de détection SEQ ID N°1

Le peptide contenant au moins la SEQ ID N°1 conjugué à la biotine dilué avec du tampon de revêtement ("coating") est adsorbé sur des plaques, revêtues de streptavidine, pendant 2h à température ambiante. Après lavage, 50 µl d'antisérums (lapin) dilués au titre optimal sont ajoutés à 50 µl de standard (peptide synthétique dilué dans du tampon) ou à 50 µl d'échantillons à doser susceptible de contenir de la périostine et sont laissés à incuber pendant 2h à température ambiante. Après lavage, 100 µl d'anticorps secondaire couplés à la peroxydase dirigés contre les immunoglobulines de lapins sont ajoutés pendant une heure à température ambiante. Les puits sont lavés et 100 µl de substrat (TMB) sont ajoutés. La réaction est stoppée avec 100 µl d'H₂SO₄.

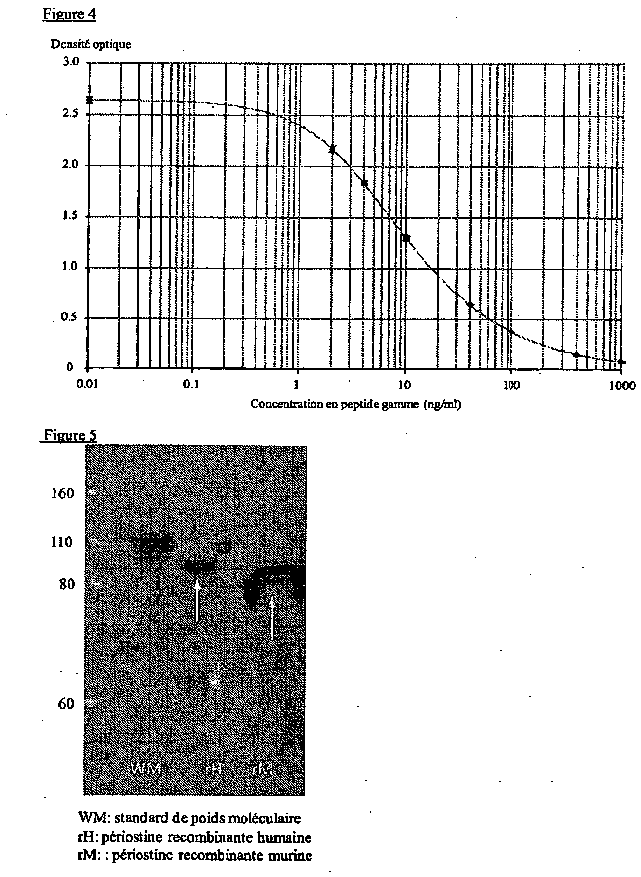

En affinant le titre par un test de déplacement, on obtient un titre de 180000 et on détermine les différents points de la gamme standard. Une courbe standard ou de calibration a été obtenue sur un graphe semi logarithmique en dosant les 7 standards (de 1,33 à 1000 ng/ml) avec l'antisérum dirigé contre la séquence SEQ ID N°1 sélectionné pour son titre (figure 4). La concentration en périostine dans les échantillons est obtenue par extrapolation de la courbe de calibration.

La détection limite de cet essai a été déterminée par le calcul de la moyenne de 20 déterminations du standard 0 auquel on retranche trois fois la déviation standard. La détection limite est de 0,39 ng/ml.

Les performances analytiques de l'ELISA ont été vérifiées en effectuant :
1) des tests de dilution : un échantillon sérique est dosé pur et dilué (cf Tableau 1). 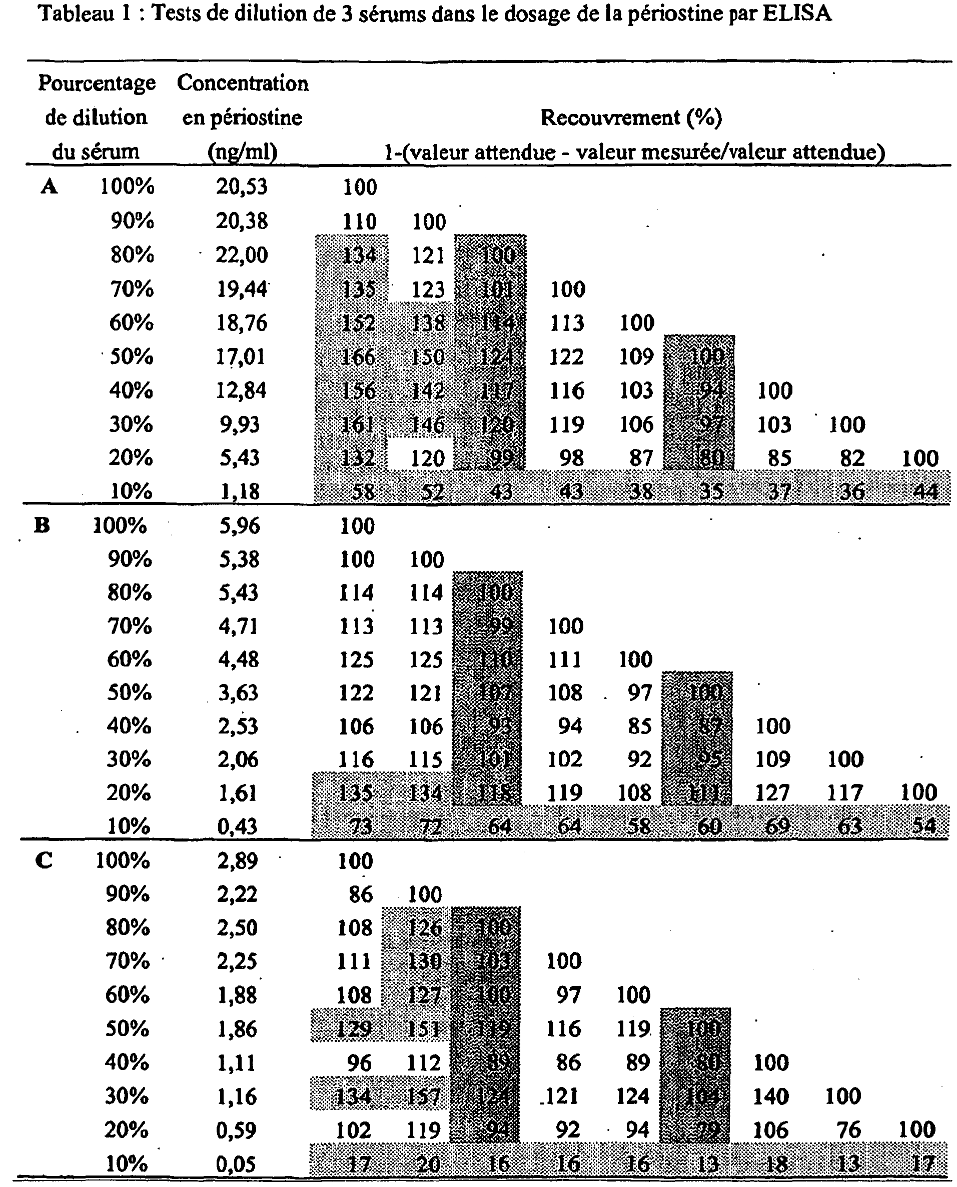 Comme le montre le tableau 1 en partant du sérum pur les dilutions ne sont pas correctes (gris clair). Pour chacun des 3 échantillons, en partant du sérum dilué à 80%, de bons recouvrements sont obtenus pour les dilutions de 10 en 10% (gris foncé). Ainsi, par soucis d'économie des échantillons, des sérums dilués à 50% soit au ½ seront utilisés et ces sérums pourront encore être dilués de 10 en 10% jusqu'à 20% soit 1/5 du sérum pur.
2) des tests de précision *intra* et *inter* essai : le même échantillon (sérum contrôle ou peptide standard) est mesuré 20 fois sur une même plaque (*intra-essai*) ou le même échantillon (sérum contrôles ou peptide standard) est mesuré dans 10 plaques différentes (*inter-essai*) afin de vérifier la variabilité des valeurs obtenues (Tableau 2 et 3).

**Tableau 2 : Précision de la mesure du peptide de gamme de séquence SEQ ID N°1 intra et inter-essai:**

| | *Intra*-essai | | *Inter*-essai | |
|---|---|---|---|---|
| Peptide gamme | Concentration en peptide périostine (ng/ml) | Coefficient de variabilité (CV), % | Concentration en peptide périostine (ng/ml) | CV, % |
| 1,33 | 1,362 ± 0,293 | 21,5 | 1,3 ± 0,25 | 19,1 |
| 2 | 2,15 ± 0,31 | 12,3 | 1,92 ± 0,25 | 12,9 |
| 4 | 3,965 ± 0,406 | 10,2 | 4,01 ± 0,52 | 13 |
| 10 | 10,045 ± 0,906 | 9 | 9,98 ± 0,7 | 7 |
| 40 | 40,607 ± 3,582 | 8,8 | 39,8 ± 2.48 | 6,2 |
| 100 | 98,197 ± 10,93 | 11,1 | 100,84 ± 8,24 | 8,2 |
| 400 | 425,426 ± 39,816 | 9,4 | 420,25 ± 37,75 | 9 |
| 1000 | 898,435 ± 77,993 | 8,7 | 901,65 ± 62,66 | 6,9 |

On constate une répétabilité correcte (CV<15%) de la mesure du peptide servant de standard pour la détermination de la concentration en périostine dans les échantillons sériques par ELISA. Seul le premier point de gamme (1,33 ng/ml) a une répétabilité moins bonne car proche de la limite de détection.

**Tableau 3 : Précision de la mesure du peptide contenant au moins la de séquence SEQ ID N°1 intra et inter-essai dans trois échantillons sériques**

| | *Intra*-essai | | *Inter*-essai | |
|---|---|---|---|---|
| Echantillons | Concentration en peptide périostine (ng/ml) | Coefficient de variabilité (CV), % | Concentration en peptide périostine (ng/ml) | CV, % |
| A | 16,123 ± 1,160 | 7,2 | 16,53 ± 1,90 | 11,47 |
| B | 4,620 ± 0,560 | 12,1 | 4,83 ± 0,62 | 12,91 |
| C | 2,838 ± 0,329 | 11,6 | 2,96 ± 0,43 | 14,62 |

On constate une répétabilité correcte de la mesure de périostine par l'ELISA pour des échantillons sériques présentant des taux élevé, moyen et bas en périostine. En effet, les CV sont inférieurs à la limite fixée de 15%, ceci que l'échantillon se situe en début, milieu ou fin de gamme.

### Exemple 3 - Analyse Western blot de la spécificité de l'antisérum contenant l'anticorps anti-périostine

Cette technique d'électrophorèse sur gel de polyacrylamide permet de séparer des protéines préalablement dénaturées, selon leurs poids moléculaire. Les échantillons sont repris dans une solution de Lithium Dodecyl Sulfate (tampon NuPAGE® LDS) et d'agent réducteur. Ils sont ensuite dénaturés à 70°C pendant 10 minutes puis déposés sur gel pour migration des protéines à 200V (180mA) pendant 40min.

Le transfert des protéines du gel de polyacrylamide sur une membrane de PolyVinyliDene Fluoride (PVDF) est suivi d'une détection des protéines immobilisées par l'antisérum spécifique de la periostine. Pour l'immunodétection, la membrane est placée dans un tampon de saturation TBS-Tween 0.1%-Lait 3% pendant une heure à température ambiante. La membrane est lavée rapidement dans du tampon de lavage puis incubée avec l'antisérum dilué au 1/1000 pendant une heure à température ambiante. Après trois lavages de 5 minutes, la membrane est incubée 1h à température ambiante en présence de l'anticorps secondaire anti-lapin dilué au 1/10000. La membrane est à nouveau rincée 3 fois 5 minutes. La membrane est incubée 1 minute dans une solution chimioluminescente (kit ECL, Amersham). Elle est exposée au contact d'un film photographique pendant un temps variable (1min-5min) dans une cassette d'autoradiographie. Le film est ensuite révélé par incubation de quelques secondes à une ou deux minutes dans du révélateur puis rincée à l'eau et fixé une minute dans du fixateur.

La figure 5 correspond à une photographie d'un western blot sur lequel les périostines recombinantes murine et humaine servent d'échantillons contrôles. Une bande pour chaque échantillon respectif est observée entre 80 et 110 kDa. La périostine ayant une taille théorique de 90kDa, on constate donc que l'antisérum contenant les anticorps anti-périostine spécifiques de l'antigène synthétique de séquence peptidique comprenant au moins une séquence de 6 à 30 acides aminés contenant au moins la séquence SEQ ID N°1, reconnait spécifiquement la périostine humaine et murine.

### Exemple 4 - Détermination du niveau de périostine sérique chez des patients atteints de cancer du sein avec métastases osseuses et des sujets contrôles

Des échantillons sériques issus de 30 patientes à jeun atteintes de cancer du sein (département de médecine de l'Institut Jules Bordet de Bruxelles) sont analysés. Avant d'effectuer les dosages, tous les échantillons sériques avaient été conservés à -70°C. Le cancer du sein a été confirmé par analyse histologique pour chacune des patientes. Parmi les 30 patientes atteintes du cancer du sein, 15 présentent des métastases osseuses visibles en radiologie. Les patientes étaient sous traitement antinéoplasique stable depuis au moins 4 semaines et n'ont pas reçu de traitement aux bisphosphonates au cours des 4 mois précédents. Les patientes n'ont pas d'antécédents d'autres maladies osseuses métaboliques. Les taux de périostine sérique ont aussi été mesurés chez 18 femmes saines et non traitées (âge moyen 53 ans, répartition entre 46 et 60 ans) recrutées à partir d'un programme de don du sang et ne présentant pas d'antécédents de maladie du sein ou de maladies osseuses métaboliques. Aucun des contrôles sains n'ont eu de traitement qui pourrait interférer avec le métabolisme osseux, incluant l'hormonothérapie substitutive chez les femmes ménopausées.

L'âge médian des patientes présentant un cancer du sein avec ou sans métastases osseuses est respectivement de 58 et 66 ans, elles sont positives aux récepteurs aux oestrogènes respectivement dans 40% et 87% des cas. Ces patientes présentent majoritairement des carcinomes mammaires de type ductal (67%). Pour les patientes présentant un cancer du sein avec métastases osseuses, le type de métastases osseuses se réparti également entre le type lytique, blastique et mixte avec une charge métastatique ≥5 dans 67% des cas.

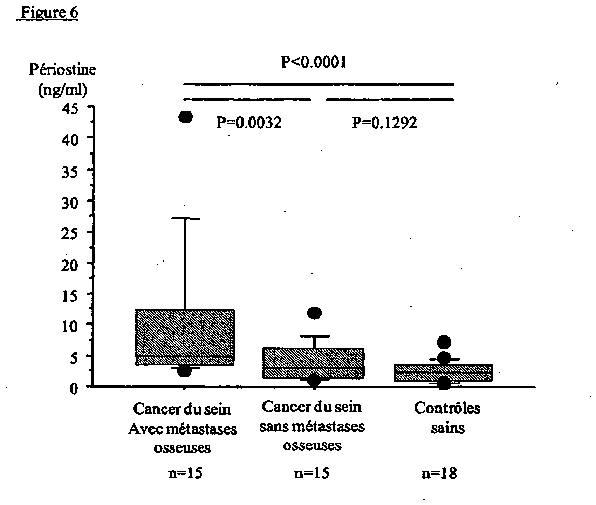

La figure 6 correspond à un graphique illustrant les résultats d'une étude clinique sur des individus sains et des individus atteints de cancer du sein présentant ou pas des métastases osseuses, à l'aide du marqueur périostine via la séquence de détection SEQ ID N°1 selon l'invention. On constate que le taux de périostine sérique moyen détecté est significativement plus élevé chez les individus métastasés (5,16 ± 15,12 ng/ml) que chez les individus sans métastases osseuses (3,14 ± 4,84 ng/ml) ou sains (2,37 ± 2,56 ng/ml). Ceci montre que la périostine globale sérique via la séquence de détection SEQ ID N°1 selon l'invention est un bon indicateur de la présence des métastases osseuses du cancer du sein.

### Exemple 5 : Expression de la périostine dans un environnement de métastases osseuses provenant de cancer du sein.

L'expression de la périostine est évaluée par PCR et par ELISA avec l'anticorps selon la présente invention dirigé contre la SEQ ID N° 1 dans plusieurs lignées cellulaires humaines de cancer du sein et de prostate qui métastase dans l'os. Les cellules MDA-B02 du cancer du sein sont ensuite injectées dans l'artère de la queue de souris recevant de l'acide zolédronique (biphosphonate) ou un placébo. L'expression de la périostine est déterminée par immunohistochimie (IHC) et PCR quantitative. La périostine sérique et les marqueurs osseux classiques sont également mesurés dans les souris. Les niveaux de périostine sérique sont mesurés chez 30 patientes atteintes de cancer du sein avec métastases osseuses (n=15) ou non (n=15) et chez 16 patientes saines.

Aucune des lignées cellulaires humaines cultivées in vitro n'exprime ou ne sécrète la périostine. La périostine murine -mais non humaine- est cependant exprimée de façon marquée dans le microrenvironnement osseux des souris ayant reçu une injection de cellules MDA-B02 par rapport aux souris contrôle. Les cellules murines stromales secrétent donc de la périostine en réponse à la présence des cellules tumorales humaines injectées. L'immunohistochimie montre que la périostine est localisée dans les cellules stromales de la moëlle osseuse. Grâce à la radiographie et les marqueurs biochimiques classiques du renouvellement osseux, on constate que le traitement à l'acide zolédronique diminue de façon marquée les lésions osseuses mais il a seulement un effet faible sur l'expression de l'ARNm de la périostine et les taux circulants. Ceci montre que l'expression de la périostine n'est pas liée au remodelage osseux en contexte métastatique. Les taux de périostine sériques sont significativement plus élevés chez les patientes atteintes d'un cancer du sein et de métastases osseuses que chez les patientes atteintes d'un cancer du sein mais sans métastases (+64 %, p=0.02) et chez les patientes saines (+117 %, p=0.01).

Par conséquent, les métastases osseuses issues du cancer du sein induisent une surexpression de périostine via les cellules stromales. Ainsi, la périostine pourrait-elle être un marqueur biochimique de la réponse précoce des cellules stromales aux métastases osseuses provenant de cancer du sein. Cette détection permettrait de détecter la présence de métastases osseuses avant la résorption de l'os et donc avant la détection possible par imagerie et avant la modification des marqueurs du remodelage osseux d'où la précocité du diagnostic possible.

### Exemple 6 - Analyse immunohistochimique de séctions d'os de souris

Les os fixés dans le paraformaldéhyde sont décalcifiés avec une solution d'Osteosoft (Merck, VWR, Val de Fontenay, France) avant déshydratation et inclusion dans de la paraffine selon les techniques usuelles de laboratoire. L'immunohistochimie a été réalisée sur des sections de 7 µm. Brièvement, les sections sont déparaffinées, les péroxydases endogènes sont bloquées et l'antigène est démasqué par une incubation de 1h à température ambiante dans du tampon TRIS-Glycine. Les sites non spécifiques sont bloqués 1h à température ambiante avec du tampon TRIS contenant 5% de sérum normal de chèvre (« normal goat sérum » NGS). Les sections sont par la suite mises à incuber durant une nuit à 4°C avec l'anticorps primaire dirigé contre la séquence SEQ ID N°1. Comme contrôle de spécificité l'anticorps primaire est préincubé 1h à 37°C avec le peptide de séquence SEQ ID N°I puis mis en contact avec les sections durant une nuit à 4°C. Après lavage, suit une incubation à température ambiante pendant 1h avec un anticorps anti IgG de lapin couplé à la peroxydase du raifort (Rabbit IgG HRP-linked Whole Ab; Source: Donkey ; GE Healthcare). Enfin, la peroxydase est mise en présence de DAB (diaminobenzidine) qui donne un marquage brun aux sites immunoréactifs. Une contre coloration est réalisée avec l'hematoxyline de Mayers qui colore les noyaux des cellules en bleu.

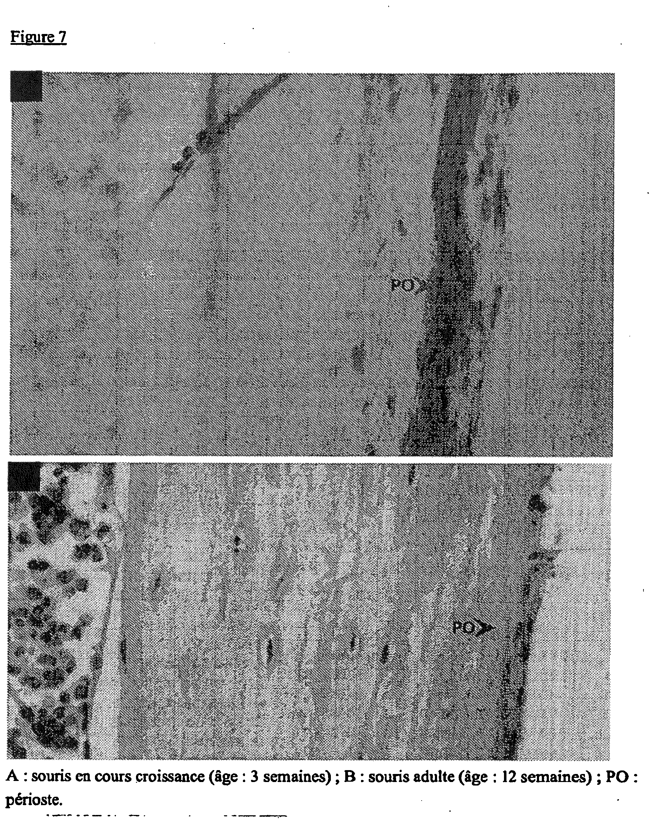

La figure 7 montre une analyse immunohistochimique réalisée avec l'anticorps spécifique de la SEQ ID N°1 de coupes de tissus osseux. Lorsque des sections d'os de souris en cours de croissance sont mises à incuber en présence de l'antisérum spécifique, une coloration apparait au niveau du périoste (cf. fig. 7A). Au cours de la croissance, l'activité d'apposition périostée (qui permet la croissance radiale de l'os par « ossification intramembranaire ») diminue. Notre analyse montre que le périoste des souris adultes est plus fin et non marqué par l'anticorps spécifique de la SEQ ID N°1 (cf. fig. 7B). Cela suggère que la SEQ ID N°1 est un marqueur de l'ossification intramembranaire et de l'apposition périostée.

## Revendications

1. Séquence peptidique SP de détection de la périostine, **caractérisée en ce qu'**elle est constituée d'au plus 30 acides aminés et inclut la séquence peptidique SEQ ID N°1, SEQ ID N°2 ou SEQ ID N°3 ou une séquence peptidique présentant un degré d'homologie supérieur ou égal à 80%, de préférence supérieur ou égal à 85% vis-à-vis de la séquences SEQ ID N°1, SEQ ID N°2 et SEQ ID N°3.

2. Séquence nucléique codant la séquence de détection SP telle que définie dans la revendication 1.

3. Moyens de détection de la périostine, **caractérisés en ce qu'**ils comprennent un outil de reconnaissance spécifique d'une ou plusieurs séquences peptidiques SP selon la revendication 1; cet outil comportant au moins un anticorps anti-périostine et/ou au moins un fragment fonctionnel dudit anticorps anti-périostine, spécifiques de SEQ ID N°1, SEQ ID N°2, SEQ ID N°3 ou d'une séquence peptidique présentant un degré d'homologie supérieur ou égal à 80%, de préférence supérieur ou égal à 85% vis-à-vis d'au moins l'une des séquences SEQ ID N°1, SEQ ID N°2 et SEQ ID N°3.

4. Procédé de production de moyens de détection de la périostine, en particulier d'Ac anti-périostine, selon la revendication 3, **caractérisé en ce qu'**il consiste essentiellement à :
a. mettre en oeuvre au moins un antigène représenté par une séquence peptidique SP telle que définie dans la revendication 1,
b. coupler ledit antigène à au moins une molécule porteuse,
c. récupérer du sang/plasma/sérum d'un animal auquel a été préalablement injecté le couple antigène-molécule porteuse,
d. sélectionner les anticorps spécifiques de SP en mettant le sérum/plasma récupéré à l'étape d) avec des antigènes SP,
e. éventuellement purifier les anticorps.

5. Procédé de détection et éventuellement de dosage de la périostine totale dans un échantillon biologique, **caractérisé en ce qu'**il consiste essentiellement à :
I. mettre en oeuvre un échantillon biologique,
II. mettre en présence ledit échantillon biologique avec des moyens de détection selon la revendication 3,
III. mettre en évidence les éventuels complexes *Ac anti-périostine*/*SP* formés;
IV. éventuellement doser ces complexes *Ac anti-périostine*/*SP* formés pour en déduire la concentration en périostine dans l'échantillon.

6. Procédé selon la revendication 5, **caractérisé en ce qu'**il comprend une étape (iv) qui consiste en un dosage ELISA, de préférence un dosage ELISA dit « par compétition » ou « sandwich ».

7. Procédé selon la revendication 5 ou 6 **caractérisé en ce que** la périostine à détecter et à doser est la périostine sous-carboxylée (*psc*), à distinguer de la périostine carboxylée (*pc*) et **en ce qu'**il consiste essentiellement à:
I*^{psc}.*- mettre en oeuvre un échantillon biologique,
I*^{psc}.*1- mettre en présence dans un milieu donné ledit échantillon biologique avec un support apte à fixer la *pc* de préférence à la *psc* ou inversement, ledit support étant :
• avantageusement un support ayant une affinité vis-à-vis du *pc* supérieure à celle vis-à-vis du *psc,*
• et, de manière plus avantageuse, un support comprenant de l'hydroxyapatite,
• et, de manière encore plus avantageuse, un support comprenant de la matrice minérale osseuse,
I*^{psc}*.2*-* éventuellement agiter ledit milieu,
I*^{psc}*.3- une fois la fixation effectuée, séparer (de préférence par gradient de densité, et, plus préférentiellement encore, par centrifugation) le support de fixation chargé en *pc* ou en *psc* (de préférence en *pc*) du reste du milieu,
II*^{psc}*. mettre en présence le support de fixation chargé en *pc* ou en *psc* (de préférence en *pc*) ou le reste du milieu avec des moyens de détection selon la revendication 3, cette dernière alternative étant préférée,
III*^{psc}*. mettre en évidence les éventuels complexes *Ac anti-périostine*/*SP* formés;
IV*^{psc}*. doser ces complexes *Ac anti-périostine*/*SP* formés pour en déduire la concentration en périostine *pc* dans le support de fixation ou en périostine *psc* dans le reste du milieu, cette dernière alternative étant préférée, et *in fine* dans l'échantillon.

8. Procédé selon la revendication 7 **caractérisé en ce que** la périostine à détecter et à doser est la périostine carboxylée (*pc*) et ce qu'il consiste essentiellement à:
1. mettre en oeuvre un échantillon biologique,
2. doser la périostine totale dans cet échantillon biologique,
3. doser la périostine *psc* dans cet échantillon biologique,
4. faire la différence entre la concentration en périostine totale et la concentration en périostine *psc* dans cet échantillon biologique, de façon à obtenir la concentration en périostine *pc* dans cet échantillon biologique.

9. Procédé de mise en évidence et d'évaluation d'un phénomène biologique non pathologique impliquant la périostine, ou de diagnostic, de suivi ou de pronostic d'une pathologie impliquant la périostine, ou de détermination de l'efficacité d'un médicament adapté au traitement d'une pathologie impliquant la périostine, **caractérisé en ce qu'**il consiste à mettre en oeuvre le procédé selon au moins l'une des revendications 5 à 8.

10. Procédé selon la revendication 5 et éventuellement au moins l'une des revendications 6 à 9 **caractérisé en ce qu'**au terme de l'analyse quantitative (étapes I-II-III-IV) in vitro de la périostine présente dans un échantillon biologique d'un sujet, on compare la concentration en périostine mesurée dans l'échantillon avec une concentration de référence correspondant à l'existence ou à l'absence de pathologie impliquant la périostine chez un sujet témoin de la même espèce.

11. Procédé selon la revendication 9 de mise en évidence et d'évaluation d'un phénomène biologique non pathologique impliquant la périostine, **caractérisé en ce que** ledit phénomène est choisi dans le groupe de phénomènes comprenant:
l'activité métabolique de l'os, en particulier du périoste, le remodelage de l'os et/ou du cartilage articulaire en fonction de l'âge, du changement hormonal, de l'activité physique, l'ossification intramembranaire, le phénomène de minéralisation vasculaire.

12. Procédé selon la revendication 9 de diagnostic, de suivi ou de pronostic d'une pathologie impliquant la périostine, ou de détermination de l'efficacité d'un médicament adapté au traitement d'une pathologie impliquant la périostine, **caractérisé en ce que** cette pathologie est choisie dans le groupe de pathologies comprenant:
• les pathologies osseuses de préférence dans le sous-groupe comprenant l'ostéoporose, les métastases osseuses du cancer du sein et de la prostate et d'autres origines, le myélome,
• et les pathologies ostéoarticulaires de préférence dans le sous-groupe comprenant l'arthrose, la polyarthrite rhumatoïde, la spondylarthrite, les fibroses notamment hépatique, et les maladies cardiovasculaires.

13. Nécessaire de détection et de dosage in vitro de la périostine, dans un échantillon biologique, **caractérisé en ce qu'**il comprend des moyens de détection de la périostine selon la revendication 3, des réactifs et du matériel pour la mise en évidence, voire le dosage, des éventuels complexes *Ac anti-périostine*/*SP* formés, selon les étapes III, voire IV, du procédé selon la revendication 5, et une notice de mise en oeuvre.

14. Nécessaire de détection et de dosage de la périostine sous-carboxylée *psc,* **caractérisé en ce qu'**il comprend des moyens de détection de la périostine selon la revendication 3, des réactifs et du matériel pour la mise en évidence, voire le dosage, des éventuels complexes *Ac anti-périostine*/*SP* formés selon les étapes III, voire IV, du procédé selon la revendication 5, un support pour la mise en oeuvre des étapes I*^{psc}*.et II*^{psc}* du procédé selon la revendication 7, c'est-à-dire un support apte à fixer la *pc* de préférence à la *psc* ou inversement, ledit support étant :
• avantageusement un support ayant une affinité vis-à-vis du *pc* supérieure à celle vis-à-vis du *psc,*
• et, de manière plus avantageuse, un support comprenant de l'hydroxyapatite,
• et, de manière encore plus avantageuse, un support comprenant de la matrice minérale osseuse comprenant de l'hydroxyapatite sous forme de cristaux.
et une notice de mise en oeuvre.

15. Anticorps anti-périostine, ou un fragment fonctionnel dudit anticorps anti-périostine, **caractérisé en ce qu'**il est spécifique de la séquence peptidique SEQ ID NO :1, SEQ ID NO:2, ou SEQ ID NO:3.

## Patentansprüche

1. Peptidische Sequenz SP für den Nachweis von Periostin, **dadurch gekennzeichnet, dass** sie aus mehr als 30 Aminosäuren besteht und die peptidische Sequenz SEQ ID Nr. 1, SEQ ID Nr. 2 oder SEQ ID Nr. 3 beinhaltet oder eine peptidische Sequenz, die einen Homologiegrad von gleich 80 % oder höher, vorzugsweise von gleich 85 % oder höher gegenüber mindestens einer der Sequenzen SEQ ID Nr. 1, SEQ ID Nr. 2 und SEQ ID Nr. 3 aufweist.

2. Nukleinsequenz, die die Nachweissequenz SP verschlüsselt wie im Anspruch 1 definiert.

3. Mittel für den Nachweis von Periostin, die **dadurch gekennzeichnet sind, dass** sie ein Werkzeug zur spezifischen Erkennung von einer oder mehreren peptidischen Sequenzen SP nach Anspruch 1 umfassen; dieses Werkzeug beinhaltet mindestens einen Antikörper Antiperiostin und/oder mindestens ein funktionelles Fragment des besagten Antikörpers Antiperiostin, die spezifisch sind für SEQ ID Nr. 1, SEQ ID Nr. 2, SEQ ID Nr. 3 oder eine peptidische Sequenz, die einen Homologiegrad von gleich 80 % oder höher, vorzugsweise von gleich 85 % oder höher gegenüber mindestens einer der Sequenzen SEQ ID Nr. 1, SEQ ID Nr. 2 und SEQ ID Nr. 3 aufweist.

4. Herstellungsverfahren von Mitteln zum Nachweis von Periostin, insbesondere Antikörper Antiperiostin, nach Anspruch 3, **dadurch gekennzeichnet, dass** es im Wesentlichen darin besteht:
a) mindestens ein Antigen einzusetzen, dargestellt durch eine peptidische Sequenz SP wie im Anspruch 1 definiert
b) besagtes Antigen an mindestens ein Trägermolekül zu binden
c) Blut/Plasma/Serum von einem Tier zu entnehmen, dem vorab die Verbindung aus Antigen und Trägermolekül injiziert wurde
d) die spezifischen Antikörper SP auszuwählen, indem das in Schritt c) gewonnene Serum/Plasma mit Antigenen SP zusammengebracht wird
e) die Antikörper eventuell zu reinigen.

5. Verfahren zum Nachweis und eventuell zur Bestimmung von Gesamtperiostin in einer biologischen Probe, **dadurch gekennzeichnet, dass** es im Wesentlichen darin besteht:
I. eine biologische Probe einzusetzen
II. besagte biologische Probe mit Nachweismitteln nach Anspruch 3 zusammenzubringen
III. die eventuellen gebildeten Komplexe *Antikörper Antiperiostin*/*SP* nachzuweisen
IV. diese gebildeten Komplexe *Antikörper Antiperiostin*/*SP* eventuell zu bestimmen, um daraus die Periostin-Konzentration in der Probe abzuleiten.

6. Verfahren nach Anspruch 5, **dadurch gekennzeichnet, dass** es einen Schritt (iv) umfasst, der in einem ELISA-Test besteht, vorzugsweise einen ELISA-Test mit der Bezeichnung "kompetitive Methode" oder "Sandwich-Methode".

7. Verfahren nach Anspruch 5 oder 6, **dadurch gekennzeichnet, dass** es sich bei dem nachzuweisenden und zu bestimmenden Periostin um untercarboxyliertes Periostin (*psc*) handelt, zu unterscheiden vom carboxyliertem Periostin (*pc*), und das im Wesentlichen darin besteht:
I*^{psc}*.- eine biologischen Probe einzusetzen
I*^{psc}*.1- die besagte biologische Probe in einem gegebenen Milieu mit einem Träger zusammenzubringen, der geeignet ist, das *pc* vorzugsweise vor psc oder umgekehrt zu fixieren, wobei es sich bei besagtem Träger handelt um:
• bevorzugt einen Träger, dessen Affinität gegenüber *pc* höher ist als jene gegenüber *psc*
• und auf bevorzugtere Weise einen Träger, der Hydroxylapatit enthält
• und auf noch bevorzugtere Weise einen Träger, der anorganische Knochenmatrix enthält
I*^{psc}*.2-das besagte Milieu eventuell zu schütteln
I*^{psc}*.3- nach erfolgter Fixierung den mit *pc* oder *psc* (vorzugsweise mit *pc*) aufgeladenen Fixierungsträger vom restlichen Milieu zu trennen (vorzugsweise durch Dichtheitsgradienten und noch bevorzugter durch Zentrifugation)
II*^{psc}*. den mit *pc* oder *psc* (vorzugsweise mit *pc*) aufgeladenen Fixierungsträger oder das restliche Milieu mit Mitteln zum Nachweis nach Anspruch 3 zusammenzubringen, wobei diese letztere Alternative bevorzugt wird
III^{psc}. die eventuellen gebildeten Komplexe *Antikörper Antiperiostin*/*SP* nachzuweisen
IV*^{psc}*. diese gebildeten Komplexe *Antikörper Antiperiostin*/*SP* zu bestimmen, um daraus die Periostin-Konzentration *pc* im Fixierungsträger oder die Periostin-Konzentration *psc* im restlichen Milieu, wobei diese letztere Alternative bevorzugt wird, und letztlich in der Probe abzuleiten.

8. Verfahren nach Anspruch 7, **dadurch gekennzeichnet, dass** es sich bei dem nachzuweisenden und zu bestimmenden Periostin um carboxyliertes Periostin (*pc*) handelt, und das im Wesentlichen darin besteht:
1. eine biologische Probe einzusetzen
2. das Gesamtperiostin in dieser biologischen Probe zu bestimmen
3. das *psc*-Periostin in dieser biologischen Probe zu bestimmen
4. zwischen der Konzentration an Gesamtperiostin und der Konzentration an *psc*-Periostin in dieser biologischen Probe zu unterscheiden, um die Konzentration an *pc*-Periostin in dieser biologischen Probe zu erhalten.

9. Verfahren zum Nachweis und zur Bewertung eines nicht pathologischen biologischen Phänomens, das Periostin impliziert, oder zur Diagnose, zur Nachverfolgung oder zur Prognose einer Pathologie, die Periostin impliziert, oder zur Bestimmung der Wirksamkeit eines geeigneten Medikaments zur Behandlung einer Pathologie, die Periostin impliziert, **dadurch gekennzeichnet, dass** es darin besteht, das Verfahren nach mindestens einem der Ansprüche 5 bis 8 durchzuführen.

10. Verfahren nach Anspruch 5 und eventuell mindestens nach einem der Ansprüche 6 bis 9, **dadurch gekennzeichnet, dass** am Ende der quantitativen in-vitro-Analyse (Schritte I-II-III-IV) des in einer biologischen Probe eines Gegenstandes vorhandenen Periostins ein Vergleich erfolgt zwischen der Konzentration an dem in der Probe gemessenen Periostin und einer Referenzkonzentration, die auftretender oder fehlender Pathologie, die Periostin bei einem Kontrollgegenstand der gleichen Art impliziert, entspricht.

11. Verfahren nach Anspruch 9 zum Nachweis und zur Bewertung eines nicht pathologischen biologischen Phänomens, das Periostin impliziert, **dadurch gekennzeichnet, dass** das besagte Phänomen aus der Gruppe von Phänomenen ausgewählt wird, die Folgendes umfasst:
Stoffwechselaktivität des Knochens, insbesondere der Knochenhaut, Remodellierung des Knochens und/oder des Gelenkknorpels in Abhängigkeit vom Alter, von hormoneller Veränderung und von körperlicher Betätigung, intramembrane Knochenbildung, Phänomen von Gefäßmineralisierung.

12. Verfahren nach Anspruch 9 zur Diagnose, zu Nachverfolgung oder zur Prognose einer Pathologie, die Periostin impliziert, oder zur Bestimmung der Wirksamkeit eines geeigneten Medikaments zur Behandlung einer Pathologie, die Periostin impliziert, **dadurch gekennzeichnet, dass** diese Pathologie aus der Gruppe von Pathologien ausgewählt wird, die Folgendes umfasst:
• die Knochenpathologien vorzugsweise in der Untergruppe, die Osteoporose, Knochenmetastasen des Brust- und Prostatakrebses und anderen Ursprungs, Myelom umfasst
• die Knochen-Gelenk-Pathologien vorzugsweise in der Untergruppe, die Arthrose, rheumatische Polyarthritis, Spondylarthritis, Fibrosen insbesondere der Leber und kardiovaskuläre Krankheiten umfasst.

13. Set zum Nachweis und zur in-vitro-Bestimmung von Periostin in einer biologischen Probe, **dadurch gekennzeichnet, dass** es Mittel zum Nachweis von Periostin nach Anspruch 3, Reagenzen und Material zum Nachweis und sogar zur Bestimmung von eventuellen gebildeten Komplexen *Antikörper Antiperiostin*/*SP* nach den Schritten III und sogar IV des Verfahrens nach Anspruch 5 und eine Durchführungsanleitung umfasst.

14. Set zum Nachweis und zur Bestimmung von untercarboxyliertem Periostin (psc), **dadurch gekennzeichnet, dass** es Mittel zum Nachweis von Periostin nach Anspruch 3, Reagenzen und Material zum Nachweis und sogar zur Bestimmung von eventuellen gebildeten Komplexen *Antikörper Antiperiostin*/*SP* nach den Schritten III und sogar IV des Verfahrens nach Anspruch 5 umfasst sowie einen Träger für die Durchführung der Schritte I*^{psc}*. und II*^{psc}* des Verfahrens nach Anspruch 7, das heißt, ein zur Fixierung von *pc* vorzugsweise vor *psc* oder umgekehrt geeigneter Träger, wobei es sich bei besagtem Träger handelt um:
• bevorzugt einen Träger, dessen Affinität gegenüber *pc* höher ist als jene gegenüber *psc*
• und auf bevorzugtere Weise einen Träger, der Hydroxylapatit enthält
• und auf noch bevorzugtere Weise einen Träger, der anorganische Knochenmatrix enthält, die Hydroxylapatit in Form von Kristallen enthält,
und eine Durchführungsanweisung.

15. Antikörper Antiperiostin oder ein funktionelles Fragment des besagten Antikörpers Antiperiostin, **dadurch gekennzeichnet, dass** es spezifisch für die peptidische Sequenz SEQ ID Nr. 1, SEQ ID Nr. 2, oder SEQ ID Nr. 3.

## Claims

1. Peptide sequence PS for detecting periostin, **characterized in that** it is constituted by 30 amino acids at most and includes the peptide sequence SEQ ID No.1, SEQ ID No.2 or SEQ ID No.3 or a peptide sequence having a degree of homology greater than or equal to 80%, preferably greater than or equal to 85%, with at least one of the sequences SEQ ID No.1, SEQ ID No.2 and SEQ ID No.3.

2. Nucleic acid sequence encoding the detection sequence PS as defined in claim 1.

3. Means for detecting periostin, **characterized in that** they comprise a recognition tool specific to one or more peptide sequences PS according to claim 1; this tool comprising at least one anti-periostin antibody and/or at least a functional fragment of said anti-periostin antibody, specific to SEQ ID No.1, SEQ ID No.2, SEQ ID No.3 or to a peptide sequence having a degree of homology greater than or equal to 80%, preferably greater than or equal to 85%, with at least one of the sequences SEQ ID No.1, SEQ ID No.2 and SEQ ID No.3.

4. Method for producing means for detecting periostin, in particular anti-periostin antibody, according to claim 3, **characterized in that** it essentially consists of:
a. utilizing at least one antigen represented by a peptide sequence PS as defined in claim 1,
b. coupling said antigen to at least one carrier molecule,
c. taking blood/plasma/serum from an animal which has previously been injected with the antigen-carrier molecule pair,
d. selecting the PS-specific antibodies by bringing the serum/plasma recovered in step c) together with PS antigens,
e. optionally purifying the antibodies.

5. Method for detecting and optionally assaying the total periostin in a biological sample, **characterized in that** it essentially consists of:
I. utilizing a biological sample,
II. bringing said biological sample together with detection means according to claim 3,
III. revealing any *anti-periostin antibody*/*PS* complexes formed;
IV. optionally assaying these *anti-periostin antibody*/*PS* complexes formed in order to deduce the periostin concentration in the sample.

6. Method according to claim 5, **characterized in that** it comprises a step (iv) which consists of an ELISA assay, preferably an ELISA assay known as "competitive" or "sandwich" ELISA.

7. Method according to claim 5 or 6, **characterized in that** the periostin to be detected and assayed is under-carboxylated periostin (*ucp*), to be distinguished from carboxylated periostin (*cp*) and **in that** it essentially consists of:
I*^{ucp}*.- utilizing a biological sample,
I*^{ucp}*1.- in a given medium, bringing said biological sample together with a support capable of binding the *cp* preferably to the *ucp* or vice versa, said support being:
• advantageously a support having an affinity for *cp* greater than that for *ucp,*
• and, more advantageously, a support comprising hydroxyapatite,
• and, even more advantageously, a support comprising bone mineral matrix,
I*^{ucp}.*2*-* optionally stirring said medium,
I*^{ucp}*.3- once the binding has been carried out, separating (preferably by density gradient and, even more preferably, by centrifugation) the binding support loaded with *cp* or with *ucp* (preferably with *cp*) from the rest of the medium,
II*^{ucp}.-* bringing the binding support loaded with *cp* or with *ucp* (preferably with *cp*) or the rest of the medium together with detection means according to claim 3, the latter alternative being preferred,
III*^{ucp}*.- identifying any *anti-periostin antibody*/*PS* complexes formed;
IV*^{ucp}*.- assaying these *anti-periostin antibody*/*PS* complexes formed in order to deduce therefrom the *cp* periostin concentration in the binding support or the *ucp* periostin concentration in the rest of the medium, the latter alternative being preferred, and ultimately in the sample.

8. Method according to claim 7 **characterized in that** the periostin to be detected and assayed is carboxylated periostin (*cp*) and **in that** it essentially consists of:
1. utilizing a biological sample,
2. assaying the total periostin in this biological sample,
3. assaying the *ucp* periostin in this biological sample,
4. establishing the difference between the total periostin concentration and the *ucp* periostin concentration in this biological sample, so as to obtain the *cp* periostin concentration in this biological sample.

9. Method for revealing and evaluating a non-pathological biological phenomenon involving periostin, or for the diagnosis, monitoring or prognosis of a pathology involving periostin, or for determining the efficacy of a medicinal product suitable for treating a pathology involving periostin, **characterized in that** it consists of implementing the method according to at least one of claims 5 to 8.

10. Method according to claim 5 and optionally at least one of claims 6 to 9 **characterized in that** at the end of the *in vitro* quantitative analysis (steps I-II-III-IV) of the periostin present in a biological sample from a subject, the periostin concentration measured in the sample is compared with a reference concentration corresponding to the existence or absence of pathology involving periostin in a control subject of the same species.

11. Method according to claim 9 for revealing and evaluating a non-pathological biological phenomenon involving periostin, **characterized in that** said phenomenon is chosen from the group of phenomena comprising:
metabolic activity of the bone, in particular of the periosteum, remodelling of the bone and/or of the articular cartilage as a function of age, hormonal change, physical activity, intramembranous ossification, the phenomenon of vascular mineralization.

12. Method according to claim 9 for the diagnosis, monitoring or prognosis of a pathology involving periostin, or for determining the efficacy of a medicinal product suitable for treating a pathology involving periostin, **characterized in that** this pathology is chosen from the group of pathologies comprising:
• bone pathologies preferably in the sub-group comprising osteoporosis, bone metastases in the case of breast and prostate cancer and cancer of other origins, myeloma,
• and osteoarticular pathologies preferably in the sub-group comprising arthrosis, rheumatoid arthritis, ankylosing spondylitis, fibroses, in particular hepatic, and cardiovascular diseases.

13. Kit for the *in vitro* detection and assay of the periostin in a biological sample, **characterized in that** it comprises means for detecting periostin according to claim 3, reagents and equipment for revealing, or assaying, any *anti-periostin antibody*/*PS* complexes formed, according to steps III or IV, of the method according to claim 5, and instructions for use.

14. Kit for the detection and assay of the under-carboxylated periostin *ucp,* **characterized in that** it comprises means for detecting periostin according to claim 3, reagents and equipment for revealing, or assaying, any *anti-periostin antibody*/*PS* complexes formed according to steps III or IV of the method according to claim 5, a support for the implementation of steps I*^{ucp}* and II*^{ucp}* of the method according to claim 7, i.e. a support capable of binding the *cp* preferably to the *ucp* or vice versa, said support being:
• advantageously a support having an affinity for *cp* greater than that for *ucp,*
• and, more advantageously, a support comprising hydroxyapatite,
• and, even more advantageously, a support comprising bone mineral matrix comprising hydroxyapatite in the form of crystals,
and instructions for use.

15. Anti-periostin antibody, or a functional fragment of said anti-periostin antibody, **characterized in that** it is specific to the peptide sequence SEQ ID No.1, SEQ ID No.2, or SEQ ID No.3.
